# EUROPÄISCHE PATENTSCHRIFT

(11) **EP 3 129 146 B1**
(45) Veröffentlichungstag und Bekanntmachung des Hinweises auf die Patenterteilung: **01.01.2020**
(21) Anmeldenummer: 15747355.4
(22) Anmeldetag: 08.04.2015
(51) Int. Cl.: B01L 3/14, A61B 5/15, A61B 5/154

(54) **AUFNAHMEEINHEIT ZUR AUFNAHME VON BLUT**
RECEIVER UNIT FOR RECEIVING BODY FLUID
UNITÉ DE RÉCEPTION DESTINÉS À RECEVOIR UN LIQUIDE CORPOREL

(30) Priorität: 09.04.2014 AT 502692014
(43) Veröffentlichungstag der Anmeldung: 15.02.2017
(73) Patentinhaber: Greiner Bio-One GmbH, 4550 Kremsmünster (AT)
(72) Erfinder: EBETSBERGER, Franz, A-4550 Kremsmünster (AT); MIZELLI, Maximilian, A-4551 Ried im Traunkreis (AT)
(74) Vertreter: Burger, Hannes
(86) Internationale Anmeldenummer: PCT/AT2015/050089
(87) Internationale Veröffentlichungsnummer: WO 2015/154115

(56) Entgegenhaltungen:
- WO-A1-97/12679
- WO-A1-98/51412
- WO-A1-2007/080230
- DE-A1-102005 062 052
- GB-A- 2 453 432
- US-A- 4 154 690
- US-A- 4 890 757
- US-A- 4 912 048
- US-A1- 2005 196 319
- US-A1- 2006 019 376
- US-A1- 2010 119 417

## Beschreibung

Die Erfindung betrifft eine Aufnahmeeinheit umfassend ein Blutprobenröhrchen und eine sein offenes Ende verschließende Verschlussvorrichtung, die zur Aufnahme von Blut dient, wie dies im Anspruch 1 beschrieben ist.

Zu Beginn der Herstellung von Blutsammelgefäßen wurden diese aus Glas gefertigt und auch schon mit einer Verschlussvorrichtung das offene Ende verschlossen. Der Aufnahmeraum wurde weiters evakuiert, um ein Ansaugen von Blut bei der Blutabnahme zu bewirken.

Ein gattungsgemäßes Proberöhrchen ist aus der WO 98/51412 A1 bekannt geworden. Das Probenröhrchen eine rohrförmig ausgebildete Behälterwand mit einem offenen Ende und ein mit einer hohlkegelförmig ausgebildeten Bodenwand verschlossenes Ende auf. Die Behälterwand weist eine Innenfläche sowie eine in einer Wandstärke davon distanzierte Außenfläche auf und definiert zwischen ihren Enden eine Längsachse. Das Proberöhrchen kann aus einem Kunststoffmaterial oder Glas gebildet sein. An der Innenfläche der Behälterwand sowie der Bodenwand ist ein in Richtung auf die Längsachse vorragender Verstärkungssteg angeordnet, welcher einen helixförmig ausgebildeten Längsverlauf aufweist. Das offene Ende der Behälterwand ist mit einer die Außenfläche übergreifenden Verschlusskappe mittels einer Gewindeanordnung verschließbar.

Die DE 10 2005 062 052 A1 beschreibt einen Einwegbioreaktor für die Kultivierung von Zellen in einem Nährstoffmedium, bestehend aus einem mit einem Deckel verschließbaren Reagenzbehälter. Im Reagenzbehälter ist mindestens eine der Behälterinnenwandung benachbarte Schikane vorgesehen, welche als in vertikaler Richtung von unten nach oben verlaufender Steg ausgebildet ist. Der Steg kann dabei wendelförmig ausgebildet sein. Der Reagenzbehälter weist an seinem oberen offenen Ende außenseitig ein Gewinde zur Verschraubung mit dem Deckel auf.

Aus der US 4,154 690 A sind Vorrichtungen zum Zentrifugieren von Flüssigkeiten mit unterschiedlicher Dichte, insbesondere Blut, bekannt geworden. Das Röhrchen weist eine Behälterwand mit einem offenen Ende sowie ein von einer Bodenwand bereichsweise verschlossenes Ende auf. In das offene Ende kann ein Dichtstopfen zum Verschleißen des Aufnahmeraums eingesetzt werden. Die Behälterinnenfläche der Behälterwand ist zwischen dem offenen Ende und dem verschlossenen Ende durchgehend zylindrisch mit einem gleichen oder einem vom offenen Ende hin zum verschlossenen Ende abnehmend ausgebildeten Innendurchmesser ausgebildet. Die Wandstärke der Behälterwand kann entweder ausgehend vom offenen Ende hin zum verschlossenen Ende stetig zunehmend oder zueinander gleich ausgebildet sein. Weiters können in der Behälterinnenfläche vertieft sowie spiralförmig verlaufende Nuten angeordnet sein. Zwischen den einzelnen Nuten bildet die Behälterinnenfläche der Behälterwand über die Nuten vorspringende Stege aus.

Ein anderes Blut-Trennröhrchen ist aus der WO 97/12679 A1 bekannt geworden. Das Blut-Trennröhrchen weist eine Behälterwand mit einem offenen Ende sowie ein von einer Bodenwand verschlossenes Ende auf. Die Behälterwand weist eine Innenfläche sowie eine in einer gleichen Wandstärke davon distanzierte Außenfläche auf und definiert zwischen ihren Enden eine Längsachse. Weiters ist die Behälterwand rohrförmig zwischen ihren Enden ausgebildet. An der Behälterinnenfläche sind mehrere über den Umfang verteil angeordnete und in Richtung auf die Längsachse vorragende Rippen angeordnet. In das offene Ende ist ein Dichtstopfen zum Abschluss des Aufnahmeraums eingesetzt.

Die WO 89/09735 A1 bzw. die daraus hervorgegangene EP 0 419 490 B1 sowie US 5,275,299 A beschreiben unterschiedlichste Verschlussvorrichtungen für insbesondere evakuierbare, zylinderförmige Gehäuse aus einem Kunststoffmaterial. Die Dicke der Wandstärke des Gehäuses war dabei aus Gründen der Stabilität sowie der Barriereeigenschaften mit ca. 1,0 mm und auch größer bemessen. Die Nenndimension bezogen auf den Außendurchmesser betrug von derartigen Röhrchen 13 mm, wobei die Nennlänge 75 mm oder 100 mm betragen hat. Es hat auch noch Röhrchen mit einem Nenndurchmesser von 16 mm bei einer Nennlänge von 100 mm gegeben. Als Nenndimension, Nenndurchmesser oder Nennlänge werden Abmessungen verstanden, die in etwa diesen Außenabmessungen entsprechen, wobei aufgrund der zumeist vorliegenden Konizität eine geringfügige Abnahme des Außendurchmessers ausgehend vom offenen Ende hin zum verschlossenen Ende vorliegt. Damit konnte nicht nur eine ausreichende Wärmeformstabilität sowie Barriereeigenschaft, sondern auch eine im Toleranzbereich liegende Geradheit sowie Verzugsfreiheit bei diesen doch massiveren Wandstärken der Behälterwand erzielt werden.

Der vorliegenden Erfindung liegt die Aufgabe zugrunde, bei dünnen oder sehr dünnen Wandstärken von Aufnahmebehältern aus einem Kunststoffwerkstoff zur direkten Aufnahme von Körperflüssigkeit, insbesondere Blut, eine ausreichende Formstabilität sowie Wärmeformbeständigkeit zu gewährleisten. Als dünne Wandstärke werden dabei Abmessungen von kleiner / gleich 0,8 mm verstanden.

Diese Aufgabe der Erfindung wird durch die Merkmale des Anspruches 1 gelöst. Die sich aus der Merkmalskombination dieses Anspruches ergebenden Vorteile liegen darin, dass so eine zusammengehörige Baueinheit zur Aufnahme bzw. zum Sammeln insbesondere von Blut geschaffen werden kann und dass damit direkt an jener Innenfläche, mit welcher die im Aufnahmeraum aufzunehmende Körperflüssigkeit, insbesondere das Blut, zur Aufbewahrung desselben in Kontakt kommt, eine ausreichende Verstärkungswirkung durch das Anordnen bzw. Vorsehen des zumindest einen Verstärkungsstegs erzielt werden kann. Damit wird es möglich, unter Beibehaltung der Nenn-Außendimensionen in Bezug auf Durchmesser sowie Längserstreckung eine zusätzliche Verstärkung der Behälterwand zu erzielen. Damit kann in weiterer Folge die Wandstärke zusätzlich reduziert werden, wodurch einerseits Rohstoffressourcen eingespart werden können und andererseits bei der anschließenden Vernichtung eine geringere Masse zu entsorgen ist. Je nach Anordnung und Ausrichtung des oder der Verstärkungsstege, welche auch als Verstärkungsrippen bezeichnet werden können, kann so die Festigkeit, die Geradheit sowie die Rundheit als auch die Formbeständigkeit positiv beeinflusst werden. Weiters kann durch das Vorsehen des zumindest einen Verstärkungsstegs zusätzlich das Ausmaß der inneren Oberfläche vergrößert werden, wodurch es so zu einer Vergrößerung der Reaktionsfläche im Innenraum kommt. Da zumeist die innere Oberfläche des Aufnahmebehälters mit Chemikalien beschichtet ist, kann dies aufgrund der vergrößerten Oberfläche zu einer Beschleunigung der gewünschten Reaktion führen. Damit könnte zum Beispiel die Blutgerinnung nach dem Einfüllvorgang rascher von statten gehen als dies bislang der Fall war. Darüber hinaus kann aufgrund der Volumenvergrößerung des Aufnahmeraums eine gegenüber bisherigen Nenndimensionen dazu größere Füllmenge bei gleicher Außenabmessung aufgenommen werden. Dadurch wird es aber in weiterer Folge auch noch möglich, den im Aufnahmeraum herrschenden Unterdruck absolut gesehen geringer zu wählen als bei gleichen Nenndimensionen von herkömmlichen Aufnahmeeinrichtungen.

Außerdem ist von Vorteil, dass so je nach dem Ausmaß des Überstandes die Versteifungswirkung des zumindest einen Verstärkungssteges für die Behälterwand auch bei einer Reduzierung der Wandstärke ein ausreichendes Ausmaß aufweist. Um ein einfaches Entformen des Formkerns und somit das Abziehen bzw. Abstreifen des Aufnahmebehälters vom Formkern zu ermöglichen, ist die Höhe bzw. das Ausmaß des Überstandes an den Neigungswinkel der Innenwand im Axialschnitt gesehen bezüglich der Längsachse abzustimmen. Darüber hinaus ist es von Vorteil, da so durch die relativ geringer Dicke der gewählten Wandstärke in Verbindung mit dem oder den Verstärkungsstegen an der Innenseite bzw. Innenfläche trotzdem ein Aufnahmebehälter geschaffen werden kann, welcher ausreichende Festigkeitseigenschaften aufweist. So kann mit einem geringeren Werkstoffeinsatz das Auslangen gefunden werden kann. Darüber hinaus wird so aber auch durch das vergrößerte Aufnahmevolumen die Möglichkeit geschaffen, bei gleicher Nenndimension des Aufnahmebehälters eine zu bislang standardmäßig eingesetzten Aufnahmebehälter dazu größere Füllmengen in den Aufnahmeraum einfüllen zu können. Damit kann in gewissen Fällen auch auf die nächstgrößere Nenndimension des Aufnahmebehälters verzichtet werden, da die größere Füllmenge nun auch im Aufnahmebehälter mit der kleineren Nenndimension aufgenommen werden kann. Damit kann auch eine Reduktion der Typenvielfalt im Bereich des Labors erreicht werden. Vorteilhaft ist auch dass das Ausmaß der Breite der Verstärkungsstege die Verstärkungswirkung einfach an unterschiedliche Anwendungsbedingungen anpassbar ist. So wird das Einströmen der aufzunehmenden Körperflüssigkeit, insbesondere von Blut, in den Aufnahmeraum aufgrund der ansonsten geringen Druckunterschiede dadurch erst einwandfrei bis zum Erreichen der vollständigen Füllmenge erzielt. Weiters kann durch das vergrößerte Innenvolumen des Aufnahmeraums aber auch der abgesenkte Druck geringer als bei den bislang eingesetzten Aufnahmeeinheiten gehalten werden. Damit erfolgt das Einströmen des Blutes in den Aufnahmeraum bezüglich der bislang eingesetzten Aufnahmeeinheiten langsamer, wodurch unter anderem die Hämloysenbildung reduziert bzw. überhaupt vermieden wird.

Weiters wird so entlang der Innenwand eine gleichmäßige Verstärkung bzw. Versteifung der Behälterwand erreicht. Durch den wendelförmigen, insbesondere den helixförmigen, Längsverlauf wird nicht nur über den Umfang gesehen eine Verstärkung- bzw. Versteifungswirkung erzielt, sondern auch über den Längsverlauf der gesamten Behälterwand.

Vorteilhaft ist weiters eine Ausbildung nach Anspruch 2, da dadurch eine noch gleichmäßigere Verstärkungs- bzw. Versteifungswirkung erzielbar ist. Dadurch kann mit äußerst dünnen Wandstärken das Auslangen gefunden werden, wobei durch die Mehrfachanordnung der wendelförmigen Verstärkungsstege eine zusätzlich hohe Versteifungswirkung erzielbar ist.

Durch die Ausbildung nach Anspruch 3 ist es möglich, durch die Wahl der Anzahl der an der Innenfläche angeordneten Verstärkungsstege sowie deren Anordnungsdichte die Aufnahmebehälter einfach an die unterschiedlichsten Einsatzbedingungen anpassen zu können. Je kleiner der Steigungswert gewählt wird, desto höher ist der flächenmäßige Anteil der Verstärkungsstege an der Innenfläche der Behälterwand.

Durch die Weiterbildung nach Anspruch 4 wird erreicht, dass so die Außenabmessungen bzw. Nenndimensionen der bislang eingesetzten Aufnahmebehälter unverändert beibehalten werden können und lediglich eine Adaption der Wandstärke erfolgt.

Durch die Ausbildung nach Anspruch 5 kann so ebenfalls auf unveränderte Nenndimensionen zurückgegriffen werden, ohne dass dafür Änderungen bei den standardmäßig eingesetzten Laborgeräten notwendig sind.

Nach einer vorteilhaften Weiterbildung gemäß Anspruch 6 wird so eine exakt umlaufende Dichtfläche im Bereich des offenen Endes geschaffen und dabei eine ungewollte Kanalbildung zwischen der Dichtfläche des Dichtstopfens und dem Dichtabschnitt vermieden.

Dabei erweist sich eine Ausgestaltung nach Anspruch 7 vorteilhaft, weil dadurch eine exakt ausgebildete Dichtfläche an der Innenfläche der Behälterwand geschaffen werden kann. Durch den nahezu bzw. in etwa zylindrisch ausgebildeten Dichtabschnitt kommt es so aber auch zu einer exakten radialen Abstützwirkung des in das offene Ende eingesetzten Dichtstopfens. Damit kann ein ungewolltes Herausdrücken desselben aus dem Aufnahmeraum leichter verhindert werden.

Durch die Ausbildung nach Anspruch 8 kann so ein steiferes und verformungsfesteres offenes Ende ausgebildet werden, um so eine bessere Abdichtung des Aufnahmeraums bei eingesetztem Dichtstopfen auch über einen längeren Zeitraum aufrechterhalten zu können. Weiters kann so aber bei sich in einer Wirrlage befindlichen, noch nicht verschlossenen Aufnahmebehältern während deren Förderbewegung ein Eindringen des kleiner ausgebildeten verschlossenen Endes in das offene Ende verhindert werden.

Dabei erweist sich eine Ausgestaltung nach Anspruch 9 vorteilhaft, weil dadurch der beim Zentrifugieren hoch belastete Boden des Aufnahmebehälters trotz der Wandstärkenreduktion mit einer ausreichenden Festigkeit ausgebildet werden kann.

Durch die Ausbildung nach Anspruch 10 kann durch den Aufnahmebehälter bei der Zentrifugation und der zumeist damit verbundenen Abstützung des Bodens bzw. des verschlossenen Endes an der Zentrifuge eine ungewollte Verformung sowie eine gegebenenfalls damit einhergehende Beschädigung verhindert werden.

Nach einer vorteilhaften Weiterbildung gemäß Anspruch 11 kann in Abhängigkeit von der Wanddickenzunahme auch ohne die Anordnung von zusätzlichen Verstärkungsstegen und/oder Rippen bzw. Stegen die bei der Zentrifugation auftretende Belastung ohne Beschädigung bzw. Verformung des Bodenbereichs aufgenommen werden.

Von Vorteil ist aber auch eine Ausbildung nach Anspruch 12, da so der Aufnahmebehälter einfach an unterschiedlichste Einsatzbedingungen durch die entsprechende Wahl des Kunststoffwerkstoffs abgestimmt werden kann.

Weiters kann vorgesehen sein, dass dem Kunststoffwerkstoff zumindest ein Barriereadditiv, wie z.B. anorganische Magnesium-Aluminiumsilikate, Ethylen-Vinylalkohol-Copolymere (EVOH), Polyvinylidenchlorid (PVDC), Masterbatch bzw. Scavenger zur Verbesserung der Gasbarriereeigenschaften zugesetzt ist. Gemäß dieser Ausbildung wird so bereits der Werkstoff selbst mit zumindest einem entsprechenden Additiv versetzt, um trotz der verringerten Wandstärke ausreichende Barriereeigenschaften zu schaffen. Je nach Wahl bzw. Kombination der Barriereadditive können so nicht nur die Gasbarriereeigenschaften sondern auch die Flüssigkeitsbarriereeigenschaften in entsprechender Weise beeinflusst, insbesondere verbessert, werden. Eine erhöhte Gasbarriereeigenschaft dient dazu, das zumeist im Aufnahmeraum herrschende Vakuum bzw. den Unterdruck auch über einen längeren Zeitraum bzw. eine längere Zeitdauer aufrecht erhalten zu können.

Bei der Ausbildung gemäß Anspruch 13 ist von Vorteil, da so je nach Art der gewählten Sperrbeschichtung einerseits die Barriereeigenschaften erhöht werden können oder aber andererseits dass auch die Reinheit der im Aufnahmeraum aufgenommenen Probe gewährleistet werden kann. Damit wird es möglich, noch exaktere Probenergebnisse zu erhalten, da auch von dem gewählten Werkstoff zur Bildung des Aufnahmebehälters kein direkter Kontakt zur Probe besteht bzw. auch von diesem keine ungewollten Bestandteile bzw. Weichmacher oder dergleichen in die Probe gelangen können.

Zum besseren Verständnis der Erfindung wird diese anhand der nachfolgenden Figuren näher erläutert.

Es zeigen jeweils in stark vereinfachter, schematischer Darstellung:
- Fig. 1: eine Aufnahmeeinheit mit Aufnahmebehälter und Verschlussvorrichtung, im Axialschnitt;
- Fig. 2: den Aufnahmebehälter mit den wendelförmigen Verstärkungsstegen nach Fig. 1, im Axialschnitt;
- Fig. 3: einen Verstärkungssteg des Aufnahmebehälters nach den Fig. 1 und 2, im Querschnitt sowie vergrößerter Darstellung;
- Fig. 4: eine weitere mögliche Ausführungsform eines Aufnahmebehälters, im Axialschnitt;
- Fig. 5: einen anderen, jedoch nicht vom Schutzumfang umfassten Aufnahmebehälter mit über den Innenumfang umlaufenden Verstärkungsstegen, im Axialschnitt;
- Fig. 6: einen Teilabschnitt einer möglichen Ausführungsform des Aufnahmebehälters im Bereich seines offenen Endes, im Axialschnitt und vergrößerter Darstellung;
- Fig. 7: einen Teilabschnitt einer anderen möglichen Ausführungsform des Aufnahmebehälters im Bereich seines offenen Endes, im Axialschnitt und vergrößerter Darstellung;
- Fig. 8: einen Teilabschnitt einer weiteren möglichen Ausführungsform des Aufnahmebehälters im Bereich seines verschlossenen Endes, im Axialschnitt und vergrößerter Darstellung;
- Fig. 9: eine andere mögliche Ausführungsform eines Aufnahmebehälters, im Axialschnitt.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind diese Lageangaben bei einer Lageänderung sinngemäß auf die neue Lage zu übertragen.

Der Begriff "insbesondere" wird nachfolgend so verstanden, dass es sich dabei um eine mögliche speziellere Ausbildung oder nähere Spezifizierung eines Gegenstands oder eines Verfahrensschritts handeln kann, aber nicht unbedingt eine zwingende, bevorzugte Ausführungsform desselben oder eine Vorgehensweise darstellen muss.

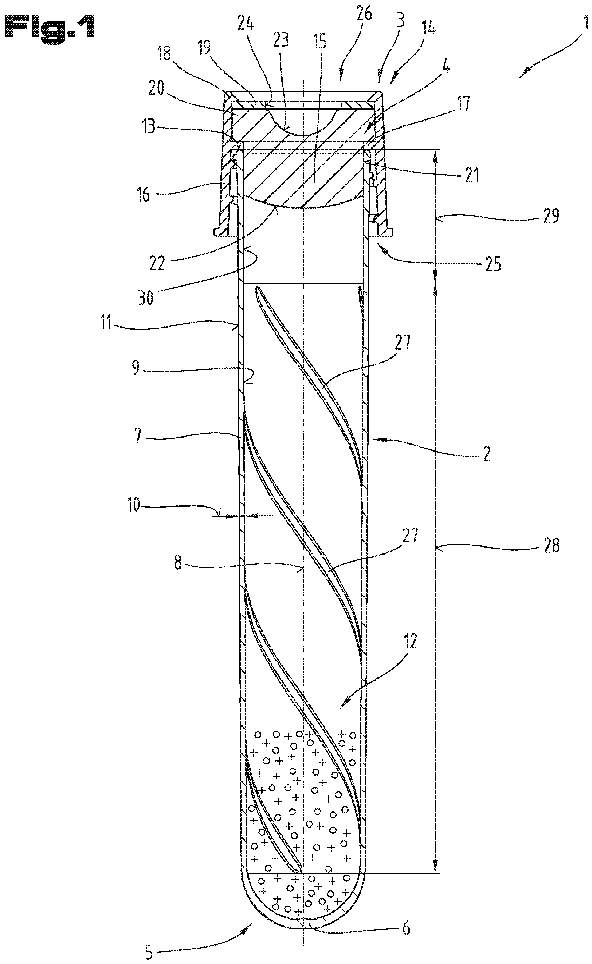

In der Fig. 1 ist eine Aufnahmeeinheit 1 z.B. für ein Gemisch aus zumindest zwei zueinander unterschiedlichen Bestandteilen bzw. Medien, wie beispielsweise Körperflüssigkeiten, oder Gewebeteilen bzw. Gewebekulturen, gezeigt, welches jedoch nicht näher bezeichnet und beschrieben wird. Bevorzugt wird die Aufnahmeeinheit 1 für das Sammeln bzw. Aufnehmen von Blut eingesetzt und kann auch als Aufnahmevorrichtung bzw. auch als Blutprobenentnahmeröhrchen bezeichnet werden.

Die Aufnahmeeinheit 1 umfasst einen in etwa zylinderförmig ausgebildeten Aufnahmebehälter 2 sowie eine Verschlussvorrichtung 3. Dieser Aufnahmebehälter 2 mit der Verschlussvorrichtung 3 wird als evakuiertes Blutprobenentnahmeröhrchen in den verschiedensten Ausführungsformen ausgebildet bzw. eingesetzt sein. Bevorzugt wird die Aufnahmeeinheit 1 mit dem zumeist evakuierten Innenraum als eine zusammengebaute Einheit dem Benutzer zur weiteren Verwendung zur Verfügung gestellt. Es wäre aber auch möglich, dass der Aufnahmebehälter 2 getrennt von der Verschlussvorrichtung 3 dem Benutzer zur Befüllung bereit gestellt wird.

Der Aufnahmebehälter 2 weist zwei voneinander distanzierten Enden 4, 5 auf, wobei bei diesem Ausführungsbeispiel das Ende 4 offen und das Ende 5 insbesondere durch eine Bodenwand 6 verschlossen ausgebildet sein kann. Allgemein kann die Bodenwand 6 auch nur als Boden bezeichnet werden. gebildet. Die Bodenwand 6 ist durch eine kalottenförmig ausgebildete Abschlusswand gebildet.

Unabhängig davon wäre es aber auch möglich, den Boden bzw. die Bodenwand 6 nicht einstückig mit dem Aufnahmebehälter 2 auszubilden, sondern das Ende 5 mit einer nicht näher beschrieben und gezeigten eigenen, weiteren Verschlussvorrichtung zum verschlossenen Ende auszubilden.

Das hier offene Ende 4 ist mit der vereinfacht dargestellten Verschlussvorrichtung 3 bedarfsweise verschließbar, wobei diese beispielsweise gemäß der EP 0 445 707 B1, der EP 0 419 490 B1, der US 5,275,299 A, der US 5,495,958 A sowie der US 5,522,518 A ausgebildet sein kann. Um Wiederholungen zu vermeiden, wird auf die Offenbarung für die Ausbildung der Kappe, der Dichtungsvorrichtung, der Kupplungsvorrichtung zwischen der Kappe und der Dichtungsvorrichtung sowie der Kappe und dem Aufnahmebehälter 2 und der Anordnung des möglichen Halterings Bezug genommen und in die gegenständliche Anmeldung übernommen.

Der Aufnahmebehälter 2 umfasst weiters eine Behälterwand 7, welche sich zwischen dem offenen Ende 4 und dem verschlossenen Ende 5 erstreckt und dabei eine Längsachse 8 definiert, wobei die Behälterwand 7 eine Innenfläche 9 sowie eine in einer Wandstärke 10 davon distanzierte Außenfläche 11 aufweist.

Die Behälterwand 7 sowie die Bodenwand 6 umgrenzen einen Aufnahmeraum 12, in welchem die Körperflüssigkeit, insbesondere das Blut, unmittelbar aufgenommen ist und mit der Innenfläche 9 in direktem Kontakt steht. Um das Einbringen zu erleichtern, bzw. überhaupt zu ermöglichen, ist der gegenüber der äußeren Umgebungsatmosphäre abgeschlossene Aufnahmeraum 12 auf einen bezüglich des Umgebungsdruckes dazu reduzierten Druck abgesenkt. Die Behälterwand 7 ist überwiegend rohrförmig ausgebildet, wobei eine geringe Konizität ausgehend vom offenen Ende 4 hin in Richtung auf das geschlossenen Ende 5 vorliegen kann. Bevorzugt wird ein Kunststoff gewählt, welcher flüssigkeitsdicht, insbesondere wasserdicht sowie gegebenenfalls gasdicht ist. Dieser kann beispielsweise aus der Gruppe von Polyethylenterephthalat (PET), Polyethylen (PE), Polypropylen (PP), Polystyrol (PS), Polycarbonat (PC), High-Density-Polyethylen (PE-HD), Acrylnitril-Butadien-Styrol-Copolymere (ABS), Polyamid (PA) bzw. einer Kombination daraus gewählt sein. Es kann aber auch Polyamid (PA) in einem Gewichtsanteil oder einem Volumsanteil von ca. 1 % bis 10 % dem Werkstoff Polyethylenterephthalat (PET) vor dem Herstellvorgang beigemischt werden.

Auf Grund der inneren Oberfläche, nämlich der Innenfläche 9, und der Behälterwand 7 mit der inneren lichten Abmessung ist somit ein innerer Querschnitt, welcher die unterschiedlichsten Querschnittsformen, wie z.B. kreisförmig, ellipsenförmig, oval, mehreckig usw., aufweisen kann, festgelegt. Die Form des äußeren Querschnittes kann auch kreisförmig, ellipsenförmig, oval, mehreckig usw. ausgebildet sein, wobei es jedoch auch möglich ist, die Form des äußeren Querschnittes unterschiedlich zur Form des inneren Querschnittes auszuführen. Bevorzugt wird jedoch ein kreisförmiger Querschnitt gewählt.

Vorteilhaft ist es, wenn die innere erste Abmessung des Aufnahmebehälters 2, ausgehend vom offenen Ende 4 hin zu dem von diesem distanzierten weiteren, verschlossenen Ende 5 sich stetig minimal verringernd zur inneren weiteren Abmessung ausgebildet ist. Dies deshalb, um beispielsweise den Aufnahmebehälter 2, wenn dieser in einem Spritzgussvorgang gefertigt ist, aus dem Spritzgusswerkzeug einfach entformen zu können. Die Verjüngung bzw. der Kegelwinkel beträgt, bezogen auf die inneren gegenüberliegenden Oberflächen des Aufnahmebehälters 2, zwischen 0,1° und 3,0°, bevorzugt zwischen 0,2° und 1,0°. An dieser Stelle sei erwähnt, dass sich die beschriebenen Abmessungen auf den Abstand zwischen den sich einander gegenüberliegenden inneren bzw. äußeren Oberflächen der Bauteile, den Durchmesser, den Umfang entlang einer Umhüllenden bzw. einer Hüll-Linie sowie den Querschnitt bzw. die Querschnittsfläche jeweils in einer der senkrecht zur Längsachse 8 ausgerichteten Ebenen sowie stets die gleiche Raumrichtung für die Ermittlung der Abmessungen beziehen können.

Wie weiters aus dieser Darstellung zu ersehen ist, weist das offenen Ende 4 eine offene Stirnseite 13 auf, welche von der bedarfsweise öffenbaren Verschlussvorrichtung 3 verschließbar ist. Dazu besteht die Verschlussvorrichtung 3 aus einer die offene Stirnseite 13 umfassenden Kappe 14 und einer darin gehalterten Dichtungsvorrichtung, wie beispielsweise einem Dichtstopfen 15, aus einem durchstechbaren, hochelastischen und selbstverschließenden Werkstoff, wie z.B. Pharmagummi, Silikonkautschuk oder Brombutylkautschuk. Diese Kappe 14 ist zumeist konzentrisch bezüglich der Längsachse 8 angeordnet und durch einen rohrförmigen oder kreisringförmig ausgebildeten Kappenmantel 16 gebildet. Zwischen der Kappe 14 und der Dichtungsvorrichtung können Mittel zum Kuppeln, wie beispielsweise Kupplungsteile einer Kupplungsvorrichtung vorgesehen sein. Bei der Kappe 20 können dies zumindest zwei über den Innenumfang bereichsweise angeordnete Fortsätze 17, 18, gegebenenfalls ein Haltering 19, und beim Dichtstopfen 15 ein zumindest bereichsweise über dessen Außenumfang vorragender Ansatz 20 sein.

Der Dichtstopfen 15 weist im vorliegenden Ausführungsbeispiel eine umlaufende und in etwa konzentrisch zur Längsachse 8 angeordnete, in etwa zylinderförmig ausgebildete Dichtfläche 21 auf, welche in ihrer in den Aufnahmeraum 12 eingesetzten, dichtenden Lage im Bereich des offenen Endes 4 an der Innenfläche 9 des Aufnahmebehälters 2 zur Anlage kommt. Dadurch soll in diesem Abschnitt die Innenfläche 9 des Aufnahmebehälters 2 in ihrer Oberflächengüte als Dichtfläche ausgebildet sein. Weiters weist der Dichtstopfen 15 eine weitere, zum Durchstich vorgesehene Dichtfläche 22 auf, welche im Zusammenwirken mit der an der Innenfläche 9 bzw. der Dichtfläche 21 anliegenden Dichtfläche den Aufnahmeraum 12 des Aufnahmebehälters 2 an dessen offener Stirnseite 13 gegenüber der äußeren Umgebung abschließt bzw. abdichtet. Durch die zumindest bereichsweise Anordnung des Fortsatzes 17 zwischen dem die Dichtfläche des Dichtstopfens 15 überragenden Ansatzes 20 und der offenen Stirnseite 13 des Aufnahmebehälters 2 kann eine Verklebung bzw. starke Anhaftung des Ansatzes 20 direkt an der Stirnseite 13 vermieden werden.

Des Weiteren kann bevorzugt der Dichtstopfen 15 auf der dem Haltering 19 zugewandten Seite eine Vertiefung 23 aufweisen, die in etwa eine gleiche Querschnittsfläche wie eine Öffnung 24 aufweist, wobei diese Öffnung 24 in ihrer Abmessung derart ausgebildet ist, dass ein ungehindertes Hindurchführen einer hier nicht dargestellten Kanüle und ein anschließendes Hindurchstechen durch die Dichtungsvorrichtung bzw. den Dichtstopfen 15 möglich ist.

Vor allem wird die Dichtheit der Verschlussvorrichtung 3 für die offene Stirnseite 13 der Aufnahmeeinheit 1 noch dadurch verbessert, wenn ein äußerer Durchmesser des Dichtstopfens 15 im Bereich seiner Dichtfläche im entspannten Zustand außerhalb des Aufnahmebehälters 2 größer ist als die innere Abmessung des Aufnahmebehälters 2 in dem dem Dichtstopfen 15 der Dichtungsvorrichtung zugewandten Bereich.

Von Vorteil ist es dabei weiters, wenn der Kappenmantel 16 als Zylinderstumpfmantel oder auch als Kegelstumpfmantel ausgebildet ist, wodurch ein Übergreifen des Kappenmantels 16 im Bereich der oberen, offenen Stirnseite 13 gewährleistet ist.

Zusätzlich zu der zuvor beschriebenen Kupplungsvorrichtung zwischen der Kappe 14 und dem Dichtstopfen 15 kann auch noch zwischen dem Aufnahmebehälter 2 und der Kappe 14 eine weitere Kupplungsvorrichtung vorgesehen sein, welche hier nur vereinfacht dargestellt worden ist. Dabei kann es sich um zusammenwirkende Vorsprünge sowie Hinterschneidungen oder aber auch um Gewindesegmente bzw. Gewindestege handeln, wie dies bereits bei derartigen Aufnahmeeinheiten üblich und bekannt ist.

So weist die Kappe 14 zwei in Richtung der Längsachse 8 voneinander distanzierte Endbereiche 25, 26 auf, wobei bei dem gezeigten Ausführungsbeispiel der offen ausgebildete Endbereich 25 über die offene Stirnseite 13 des Aufnahmebehälters 2 übergreifend angeordnet ist und die Stirnseite 13 bis nahe oder sogar anliegend an den Fortsatz 17 heranreicht. In der hier gezeigten Stellung liegt die Stirnseite 13 satt an der dieser zugewandten Oberfläche des Fortsatzes 17 an.

An der Innenfläche 9 der Behälterwand 7 ist zumindest ein in Richtung auf die Längsachse 8 vorragender Verstärkungssteg 27 angeordnet. Der zumindest eine Verstärkungssteg 27 ist bei diesem Ausführungsbeispiel zumindest an einem ersten Teilabschnitt 28 der Innenfläche 9 der Behälterwand 7 angeordnet bzw. ausgebildet, wobei sich der erste Teilabschnitt 28 ausgehend vom verschlossenen Ende 5 in Richtung auf das offene Ende 4 erstreckt. Der zumindest eine Verstärkungssteg 27 kann sich mit seinem Stegende auch noch in die in etwa kalottenförmig ausgebildete Bodenwand 6 hinein erstrecken.

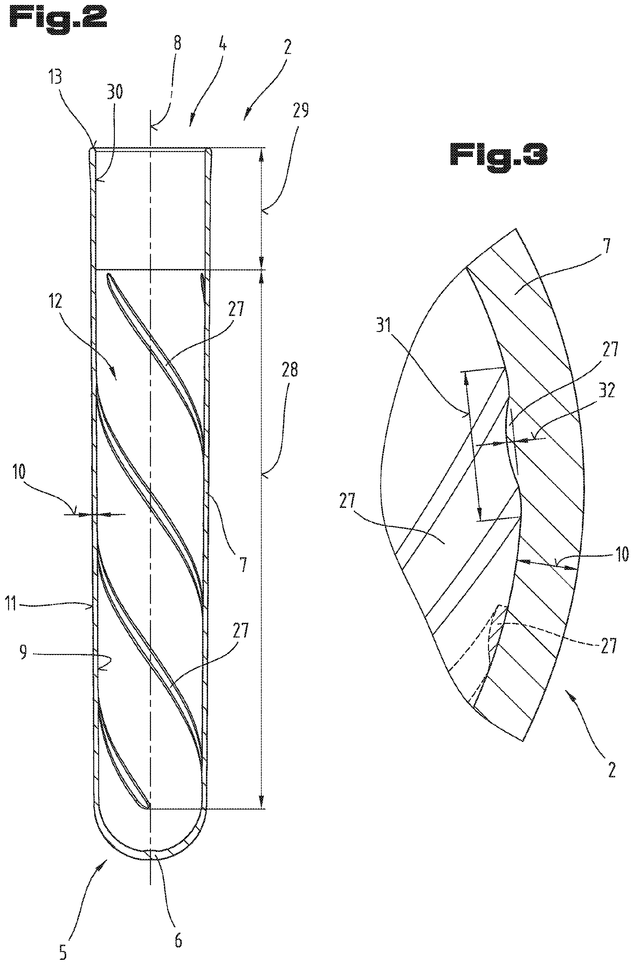

In den Fig. 2 und 3 ist der zuvor beschriebene Aufnahmebehälter 2 alleinig und somit ohne der Verschlussvorrichtung 3 dargestellt, wobei wiederum für gleiche Teile gleiche Bezugszeichen bzw. Bauteilbezeichnungen wie in der vorangegangenen Fig. 1 verwendet werden. Um unnötige Wiederholungen zu vermeiden, wird auf die detaillierte Beschreibung in der vorangegangenen Fig. 1 hingewiesen bzw. Bezug genommen.

Der hier gezeigte und beschriebene Aufnahmebehälter 2 weist an seiner Innenfläche 9 ausgehend vom verschlossenen Ende 5 hin in Richtung auf das offene Ende 4 den ersten Teilabschnitt 28 auf, an welchem der zumindest eine Verstärkungssteg 27 angeordnet bzw. ausgebildet ist. Bei diesem Ausführungsbeispiel ist noch im Bereich des offenen Endes 4 dargestellt, dass an der Innenfläche 9 der Behälterwand 7 der an den ersten Teilabschnitt 28 daran anschließende, sich hin bis zur Stirnseite 13 erstreckende zweite Teilabschnitt 29 ausgebildet bzw. vorgesehen ist. Der zweite Teilabschnitt 29 kann dabei bezüglich der Längsachse 8 bevorzugt in etwa zumindest bereichsweise zylindrisch ausgebildet sein. Dieser dient dazu, die zuvor beschriebene Dichtfläche als Dichtabschnitt 30 an der Innenfläche 9 auszubilden, an welcher der Dichtstopfen 15 mit seiner Dichtfläche 21 dichtend anliegt und den Aufnahmeraum 12 abschließt.

Der zumindest eine Verstärkungssteg 27 weist einen wendelförmigen, insbesondere helixförmigen Längsverlauf um die Längsachse 8 sowie in Richtung der Längsachse 8 gesehen auf. Bevorzugt sind jedoch mehrere über den Umfang gleichmäßig verteilt angeordnete Verstärkungsstege 27 vorgesehen. Die Anzahl der Verstärkungsstege 27 kann je nach Bedarf frei gewählt werden, wobei bei den derzeit verwendeten Nenndimensionen eine Anzahl zwischen 2 Stück und 6 Stück als vorteilhaft erscheint. Weiters kann eine Steigung des zumindest einen Verstärkungsstegs 27 aufgrund seines schraubenlinienförmigen Längsverlaufs einen Wert aufweisen, der aus einem Wertebereich stammt bzw. ausgewählt ist, dessen untere Grenze 20 mm und dessen obere Grenze 60 mm beträgt. Der Wert der Steigung ist hier auf einen vollen Umfang mit 360°bezogen. Die Wandstärke 10 wurde für die nachfolgend beschriebenen Belastungsversuche bei einer Nenndimension von 13/75 mit 0,5 mm gewählt.

Je geringer die Steigung gewählt wird, desto besser wird das Kollapsverhalten der Behälterwand bei einer länger einwirkenden Temperaturbelastung. Wird beispielsweise der Aufnahmebehälter 2 einer Temperatur von kleiner 60°C über eine Zeitdauer von 20 Stunden [h] ausgesetzt, kommt es zu keiner nennenswerten Ovalisierung des Querschnitts. Bei einem Einwirken einer Temperatur von ca. 65 °C über eine Zeitdauer von 20 Stunden [h] kommt es zu einer geringfügigen Ovalisierung, jedoch noch zu keinem völligen Kollaps. Das Ausmaß der Ovalisierung kann dabei z.B. zwischen 15 % und 20 % vom Kreisquerschnitt betragen. Wird die Temperatur auf 70 °C erhöht, wird ein vollständiger Kollaps schon nach 270 Sekunden [s] eintreten.

So wäre es aber auch möglich, beim Vorsehen von mehreren der Verstärkungsstege 27 diese mit zueinander unterschiedlichen Steigungen auszubilden, wobei dies auch zu einem gegenseitigen Schneiden der Verstärkungsstege 27 führen kann. Es wäre aber auch noch möglich, die Steigungsrichtung wie z.B. Recht- oder Linksgängig zueinander unterschiedlich zu wählen. Unabhängig davon könnte aber auch noch die gegenseitige Anordnung und Ausrichtung der Verstärkungsstege 27 so erfolgen, dass sich diese einander schneiden und im Schnittpunkt eine Art von Netz bilden.

Wie nun besser aus der Fig. 3 zu ersehen ist, kann eine Breite 31 des zumindest einen Verstärkungsstegs 27 in senkrechter Richtung bezüglich seiner Längserstreckung einen Wert aufweisen, der aus einem Wertebereich stammt, dessen untere Grenze 0,5 mm, insbesondere 1,0 mm, und dessen obere Grenze 4,0 mm beträgt. Ein Überstand 32 des zumindest einen Verstärkungsstegs 27 über die Innenfläche 9 kann einen Wert aufweisen, der aus einem Wertebereich stammt, dessen untere Grenze 0,05 mm und dessen obere Grenze 0,6 mm beträgt. Bevorzugt wird der Überstand mit einem Wert von ca. 0,2 mm bis 0,4 mm gewählt. Der Verstärkungssteg 27 kann in seinem Querschnitt gesehen, einen bogenförmigen Querverlauf aufweisen. Dieser Querverlauf könnte z.B. durch einen Abschnitt eines Kreisbogens gebildet sein und randseitig in die Innenfläche 9 übergehen.

Um eine Reduktion der Wandstärke 10 der Behälterwand 7 zu erzielen, kann diese einen Wert aufweisen, der aus einem Wertebereich stammt, dessen untere Grenze 0,3 mm und dessen obere Grenze 0,8 mm beträgt. Eine bevorzugte Wandstärke 10 für die Nenndimension 13/75 kann z.B. 0,5 mm betragen. Bei den Nenndimensionen 13/100 und 16/100 kann die Wandstärke 10 z.B. 0,7 mm betragen.

Die Bodenwand 6 kann eine ähnliche Wandstärke aufweisen, wie die Behälterwand 7 oder aber auch eine dazu größere oder kleinere Wandstärke. Aufgrund der kalottenförmigen Raumform der Bodenwand 6 wird bereits eine hohe Eigenfestigkeit in diesem Abschnitt des Aufnahmebehälters 2 erzielt. Die Festigkeit bzw. Steifigkeit der Behälterwand 7 mit der reduzierten Wandstärke 10 wird durch das Vorsehen des zumindest einen Verstärkungsstegs 27 erhöht. Damit kann bei Einsparung an Werkstoffmasse sowie bei gleicher äußerer Nenndimensionen auch im Bereich der Behälterwand 7 eine ausreichende Festigkeit sowie Stabilität erzielt werden.

Weiters soll die Behälterwand 7 mit Ausnahme des zumindest einen daran angeordneten bzw. ausgebildeten Verstärkungsstegs 27 zumindest in ihrem ersten Teilabschnitt 28 mit einer nahezu durchgängig konstanten Wandstärke 10 ausgebildet sein. Durch diese konstant dünne Wandstärke 10 kann bei einer entsprechend gegenüber bisher eingesetzten und verwendeten Aufnahmebehältern mit deren Raumform und den dabei vorliegenden unveränderten Außenabmessungen eine Erhöhung des Innenvolumens des Aufnahmebehälters erzielt werden. Dies führt weiters bei gleichem Ansaugvolumen wie bei den bisherigen Standard-Aufnahmeeinheiten dazu, dass ein geringeres Vakuum bzw. Unterdruck aufgebracht werden muss, um das gleiche Nennvolumen bei der Befüllung zu erreichen. Die Verringerung des Vakuums führt aber weiters auch noch zu einer geringeren Fließgeschwindigkeit bei der Abnahme des Blutes und damit zu einer schonenderen Behandlung. Damit treten weiters eine geringere Hämolysengefahr sowie eine Schonung der Patientenvene ein.

Weiters kommt es durch die Reduktion der Wandstärke 10 aber auch zu einer Vergrößerung der Oberfläche im Bereich des Aufnahmeraums 12. Dies schafft aber auch eine Vergrößerung der Reaktionsfläche zwischen der in den Aufnahmeraum 12 eingebrachten Körperflüssigkeit, insbesondere Blut, und möglichen auf der Innenfläche 9 üblicherweise aufgebrachten Chemikalien. Damit kann beispielsweise eine Beschleunigung der Reaktionen nach dem Einbringen des Blutes in den Aufnahmeraum 12 und der oder den dort bereits vorhandenen Chemikalien erreicht werden. So kann beispielsweise eine raschere Blutgerinnung erzielt werden.

Auch das Anbringen der Rippen bzw. Verstärkungsstege 27 vergrößert noch zusätzlich die dem Aufnahmeraum 12 zugewendete Oberfläche. Wie bereits einleitend beschrieben, sind derzeit auf dem Markt mehrere in den Abmessungen zueinander unterschiedliche Aufnahmeeinheiten, insbesondere Blutprobenröhrchen, im Einsatz. Dabei wird als Nenndimension bzw. Nenndurchmesser der Außendurchmesser des Aufnahmebehälters bevorzugt im Bereich seines offenen Endes 4 sowie die Länge des Aufnahmebehälters in Richtung seiner Längsachse 8 herangezogen. Dabei gibt es naturgemäß geringfügige Abweichungen der tatsächlichen Durchmesser sowie Längen von den nachfolgend genannten Standard-Außenabmessungen.

Eine erste mögliche Ausführungsform des Aufnahmebehälters 2 weist einen Nenndurchmesser von 13 mm bei einer Nennlänge von 75 mm auf. Eine zweite Ausführungsform weist beispielsweise bei einem Nenndurchmesser von 13 mm eine Nennlänge von 100 mm auf. Es gibt aber auch Aufnahmebehälter mit einem Nenndurchmesser von 16 mm bei einer Nennlänge von 100 mm.

Damit kann eine Gesamtlänge des Aufnahmebehälters 2 in Richtung seiner Längsachse 8 einen Wert aufweisen, der aus einem Wertebereich stammt, dessen untere Grenze 65 mm und dessen obere Grenze 130 mm beträgt. Des Weiteren kann ein Außendurchmesser des Aufnahmebehälters 2 im Bereich seines offenen Endes 4 einen Wert aufweisen, der aus einem Wertebereich stammt, dessen untere Grenze 12 mm und dessen obere Grenze 18 mm beträgt.

Weiters ist hier noch zu ersehen, dass der zumindest eine Verstärkungssteg 27 ausschließlich im ersten Teilabschnitt 28 der Innenfläche 9 der Behälterwand 7 angeordnet bzw. ausgebildet ist.

Betrachtet bzw. vergleicht man nun einzelne zuvor beschriebene bislang eingesetzte Standard-Aufnahmeeinheiten, so kann beispielsweise bei einer Nenndimension 13/75 und der dabei üblicherweise verwendeten Wandstärke von ca. 1,0 mm bis 1,1 mm bei Reduktion der Wandstärke 10 auf einen Wert von 0,8 mm die Oberfläche um ca. 6 % vergrößert werden. Wird die Wandstärke 10 noch weiter reduziert und beträgt diese beispielsweise nur noch 0,5 mm, wobei zusätzlich noch die in der Fig. 2 dargestellten Verstärkungsstege 27 daran angeordnet bzw. ausgebildet sind, kann dies zu einer Vergrößerung der Oberfläche bezüglich des bekannten Aufnahmebehälters mit der Nenndimension 13/75 von ca. 12 % führen.

Durch die Vergrößerung des Aufnahmeraums 12 aufgrund der Verringerung der Wandstärke 10 führt dies aber auch bei zueinander gleichen Füllmengen bzw. Aufnahmevolumen zwischen den bislang verwendeten Standard-Aufnahmeeinheiten und jenen Aufnahmeeinheiten 1 mit dazu verringerter Wandstärke sowie dem zumindest einen an der Innenfläche 9 angeordneten Verstärkungssteg 27 zu einem bei vertikaler Lage niedrigeren Flüssigkeitsspiegel.

Durch das gegenüber bisher eingesetzten Standarddimensionen vergrößerte Aufnahmevolumen wird es dann auch noch möglich, anstatt der bisher verwendeten Nenndimension 13/100 auf die nun dazu in der Länge kleinere Nenndimension 13/75 mit dem dazu vergrößerten Aufnahmevolumen zu wechseln. Weiters wäre es aber auch möglich, bei einigen Füll- bzw. Abnahmemengen anstatt der Nenndimension 16/100 auf die einen geringeren Außendurchmesser aufweisende Nenndimension 13/100 zu wechseln, da durch die Vergrößerung des Aufnahmevolumens nun auch eine größere Füllmenge weitgehend in der Nenndimension 13/100 aufgenommen werden kann. Dies deshalb, da der Aufnahmeraum 12 und damit das Aufnahmevolumen des neu geschaffenen Aufnahmebehälters 2 das Einbringen einer dazu größeren Füllmenge erlaubt.

Damit kann aber auch das max. Aufnahmevolumen bei der Nenndimension 13/100 mit der reduzierten Wandstärke 10 und dem zumindest einen Verstärkungssteg 27 z.B. 7 ml betragen.

Darüber hinaus kann aber auch eine Reduktion des Raumbedarfs beim Transport sowie der Lagerung erzielt werden, wodurch eine höhere Menge bzw. Anzahl an Probenmaterial auf gleichem Raum gelagert werden kann. Ein wesentlicher Faktor spielt aber auch die Reduzierung der Entsorgungskosten für den Endverbraucher, da diese zumeist massen- bzw. gewichtsabhängig berechnet werden.

Betrachtet man nun die bislang verwendeten Aufnahmeeinheiten mit der Nenndimension 13/75 sowie die neu geschaffenen Aufnahmeeinheiten 1 der gleichen Nenndimension 13/75 jedoch mit der dazu reduzierten Wandstärke 10, wird sich bei gleicher Füllmenge der Abstand zwischen dem Flüssigkeitsspiegel und dem Dichtstopfen vergrößern, wodurch sich aber auch der Volumenanteil dieses Zwischenraums erhöht.

Gemäß der Norm ISO 6710 für einmal verwendbare Blutabnahmeröhrchen ist bei einer Nennfüllmenge von ≥ 0,5 ml und kleiner 5 ml ein so genannter Headspace (entspricht dem Volumen des Freiraums) von 25 % des gesamten Nenninhalts (Nennvolumen) einzuhalten. Beträgt die Füllmenge ≥ 5 ml ist ein Headspace von 15 % des gesamten Nenninhalts einzuhalten. Bei einer Reduzierung der Wandstärke 10 der Nenndimension 13/75, zum Beispiel auf 0,8 mm und ohne der Verstärkungsstege 27, kann sich der Headspace bei gleicher Füllmenge von 4 ml bereits um ca. 25 % bis 28 % erhöhen. Wird hingegen die Wandstärke 10 auf einen Wert von 0,5 mm reduziert und die Anzahl der Verstärkungsstege 27 mit vier Stück bei einem Überstand von 0,25 mm gewählt, vergrößert sich der Headspace schon um ca. 52 % bis 55 % bezüglich des mindestens einzuhaltenden Headspace. Durch diese Vergrößerung des Headspace kann aber eine größere Füllmenge aufgenommen werden, bis dass erneut der von der Norm vorgeschriebene Freiraum erhalten bleibt.

Wie bereits zuvor beschrieben, kann durch die Vergrößerung des Volumens des Aufnahmeraums 12 auch der Evakuierungsgrad gegenüber den bislang eingesetzten Nenndimensionen reduziert werden. So beträgt beispielsweise bislang bei der Nenndimension 13/75 der Evakuierungsgrad ca. 80 % bis 84 %, insbesondere 82 %. Um das gleiche Ansaugvolumen zu erreichen, kann der Evakuierungsgrad bei einer Reduzierung der Wandstärke auf 0,8 mm auf einen Wert von ca. 71 % bis 75 %, insbesondere 73 %, gesenkt werden. Wird die Wandstärke z.B. auf 0,5 mm reduziert und sind vier Stück Rippen, wie zuvor beschrieben, vorgesehen, reduziert sich der Evakuierungsgrad auf ca. 64 % bis 68 %, insbesondere 66 %. Damit kann aber durch den reduzierten Evakuierungsgrad die dafür eingesetzte Energie beim Evakuieren reduziert werden. Durch den damit verbundenen geringen Druckunterschied zwischen dem Aufnahmeraum 12 und dem äußern Umgebungsdruck, kann aber auch der Abbau des abgesenkten Innendrucks verzögert werden, wodurch eine längere Lagerdauer erzielbar ist.

Betrachtet man nun den Innendruck des Aufnahmeraums 12 von einer Aufnahmeeinheit 1 mit der Nenndimension 13/75 sowie einer Nennfüllmenge bzw. einem Nennvolumen von 4 ml bezogen auf eine Basis von 1000 mbar, so beträgt dieser bei bislang eingesetzten Standardröhrchen mit der Nenndimension 13/75 beispielsweise zwischen 180 mbar und 200 mbar, insbesondere 190 mbar. Durch die Verringerung der Wandstärke auf 0,8 mm und der damit verbundenen Volumenvergrößerung des Aufnahmeraums 12 kann der Innendruck beispielsweise um ca. 40 % bis 45 %, insbesondere um 42 % höher gewählt werden als jener relativ geringe Innendruck der ursprünglichen Nenndimension 13/75. Bei einer weiteren Reduzierung der Wandstärke 10 bei gleicher Nenndimension auf 0,5 mm und den zuvor beschriebenen vier Stück Verstärkungsstegen 27 kann sogar der Innendruck bezüglich des relativ geringen Innendrucks der ursprünglichen Nenndimension 13/75 um ca. 76 % bis 82 %, insbesondere 79 % höher gewählt werden.

Wird nun beabsichtigt ein Nennvolumen von 5 ml in ein Standardröhrchen mit der Nenndimension 13/75 einzufüllen, ist dies bei der bislang eingesetzten dickeren Wandstärke nicht möglich. Bei einer Reduzierung der Wandstärke auf 0,8 mm kann bereits ein Headspace bei einem Füllnennvolumen von 5 ml mit ca. 17 % erzielt werden. Wird hingegen die Wandstärke 10 auf 0,5 mm reduziert und vier der zuvor beschriebenen helixförmigen Verstärkungsstege 27 mit dem Überstand 32 von 0,25 mm angeordnet, kann ein Headspace von 25 % erreicht werden.

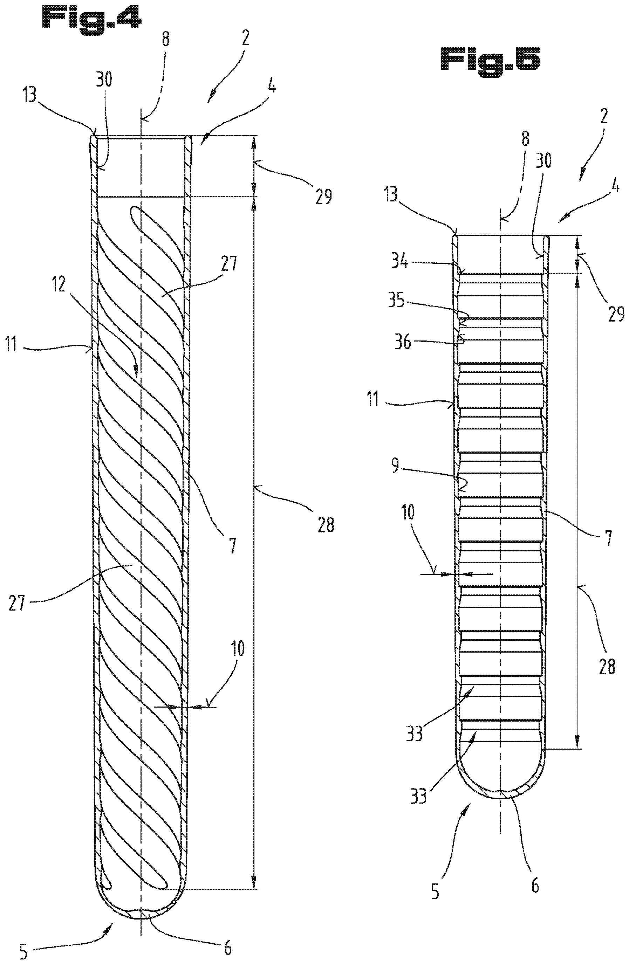

In der Fig. 4 ist eine weitere und gegebenenfalls für sich eigenständige Ausführungsform des Aufnahmebehälters 2 gezeigt, wobei wiederum für gleiche Teile gleiche Bezugszeichen bzw. Bauteilbezeichnungen wie in den vorangegangenen Fig. 1 bis 3 verwendet werden. Um unnötige Wiederholungen zu vermeiden, wird auf die detaillierte Beschreibung in den vorangegangenen Fig. 1 bis 3 hingewiesen bzw. Bezug genommen.

Der hier dargestellte Aufnahmebehälter 2 entspricht in seiner Nenndimension dem Nenndurchmesser von 13 mm sowie einer Nennlänge von 100 mm. Dieser Aufnahmebehälter 2 kann zur Bildung der Aufnahmeeinheit 1 wiederum mit der zuvor in der Fig. 1 beschriebenen Verschlussvorrichtung 3 in entsprechender Weise verschlossen werden.

Der Aufnahmebehälter 2 weist das offene Ende 4 sowie das mit der Bodenwand 6 verschlossene Ende 5 auf. Zwischen den beiden Enden 4, 5 erstreckt sich die Behälterwand 7, welche die Längsachse 8 definiert. An der Innenfläche 9 sind wiederum mehrere Verstärkungsstege 27 vorgesehen bzw. angeordnet, wobei diese wendelförmig bzw. helixförmig ausgebildet sind und über die Innenfläche 9 in Richtung auf die Längsachse 8 vorragen.

Weiters ist bei diesem Ausführungsbeispiel gezeigt, dass die Steigung der einzelnen Verstärkungsstege 27 mit 30 mm gewählt worden ist. Als Anzahl der Verstärkungsstege 27 wurden hier vier Stück gewählt, welche gleichmäßig verteilt über den Umfang angeordnet sind. Auch hier ist wiederum ein erster Teilabschnitt 28 vorgesehen, der sich ausgehend vom verschlossenen Ende 5 entlang der Behälterwand 7 hin in Richtung auf das offene Ende 4 erstreckt, jedoch noch vor der Stirnseite 13 endet. Anschließend an den ersten Teilabschnitt 28 ist der zweite Teilabschnitt 29 vorgesehen, welcher den Dichtabschnitt 30 zur Anlage des zuvor beschriebenen Dichtstopfens 15 mit seiner Dichtfläche 21 bildet.

Zur Reduzierung der Wandstärke 10 sind hier für die Erzielung der Formstabilität sowie der Warmformbeständigkeit mehrere der wendelförmig- bzw. helixförmig verlaufenden Verstärkungsstege 27 vorgesehen.

Unabhängig davon wäre es aber auch noch möglich, die Steigung der einzelnen Verstärkungsstege 27 zu der zuvor angegebenen Steigung größer oder aber auch kleiner zu wählen.

In der Fig. 5 ist eine nicht beanspruchte Ausführungsform des Aufnahmebehälters 2 gezeigt, wobei wiederum für gleiche Teile gleiche Bezugszeichen bzw. Bauteilbezeichnungen wie in den vorangegangenen Fig. 1 bis 4 verwendet werden. Um unnötige Wiederholungen zu vermeiden, wird auf die detaillierte Beschreibung in den vorangegangenen Fig. 1 bis 4 hingewiesen bzw. Bezug genommen.

Auch dieser hier dargestellte Aufnahmebehälter 2 dient wiederum zur Aufnahme von Körperflüssigkeiten, insbesondere Blut, und ist mit einer nicht näher dargestellten Verschlussvorrichtung 3 verschließbar.

Auch dieser Aufnahmebehälter 2 weist das offene Ende 4 sowie das mit der Bodenwand 6 verschlossene Ende 5 auf, wobei sich zwischen den Enden 4, 5 die Behälterwand 7 erstreckt und die Längsachse 8 definiert. An der Innenfläche 9 des Aufnahmebehälters 2 sind bei diesem Ausführungsbeispiel mehrere in Richtung der Längsachse 8 hintereinander angeordnete und voneinander distanzierte Verstärkungsstege 33 vorgesehen. Dabei sind die mehreren Verstärkungsstege 33 jeweils in einer senkrecht bezüglich der Längsachse 8 ausgerichteten Ebene zueinander angeordnet und erstrecken sich über den Umfang bevorzugt durchlaufend. Die zwischen der Innenfläche 9 und der Außenfläche 11 ausgebildete Wandstärke 10 des Aufnahmebehälters 2 kann in den zuvor beschriebenen Grenzen liegen.

Im Axialschnitt gesehen, weisen die Verstärkungsstege 33 ausgehend vom offenen Ende 4 hin auf das verschlossene Ende 5 eine Art treppenartige Ausbildung auf.

Ausgehend von der Innenfläche 9 ist eine leicht abfallend treppenförmig ausgebildete Übergangsstufe 34 vorgesehen, welche an eine in etwa zylindrisch verlaufende Stirnfläche 35 des zumindest einen Verstärkungsstegs 33 übergeht. Diese Stirnfläche 35 stellt den größten Überstand des Verstärkungsstegs 33 über die Innenfläche 9 dar. Anschließend an die Stirnfläche 35 ist ein sich vom offenen Ende 4 hin in Richtung auf das verschlossene Ende 5 erweiternder Übergangsabschnitt 36 vorgesehen, welcher in die Innenfläche 9 übergeht bzw. an dieser endet. Dabei sei erwähnt, dass die Bezeichnungen der Übergangsstufe 34, der Stirnfläche 35 sowie des Übergangsabschnitts 36 auf die Ausbildung des Verstärkungsstegs 33 im Axialschnitt betrachtet gewählt worden ist. Räumlich gesehen bildet die Stirnfläche 35 zum Beispiel eine Zylinderfläche und der Übergangsabschnitt 36 einen Kegelabschnitt aus.

Diese zuvor beschriebenen Querschnittsform des Verstärkungsstegs 33 könnte aber auch bei den in den Fig. 1 bis 4 beschriebenen Verstärkungsstege 27 mit dem helixförmigen Längsverlauf Anwendung finden, wobei dies in der Fig. 3 an der Innenfläche 9 im unteren Bereich in strichlierten Linien dargestellt ist.

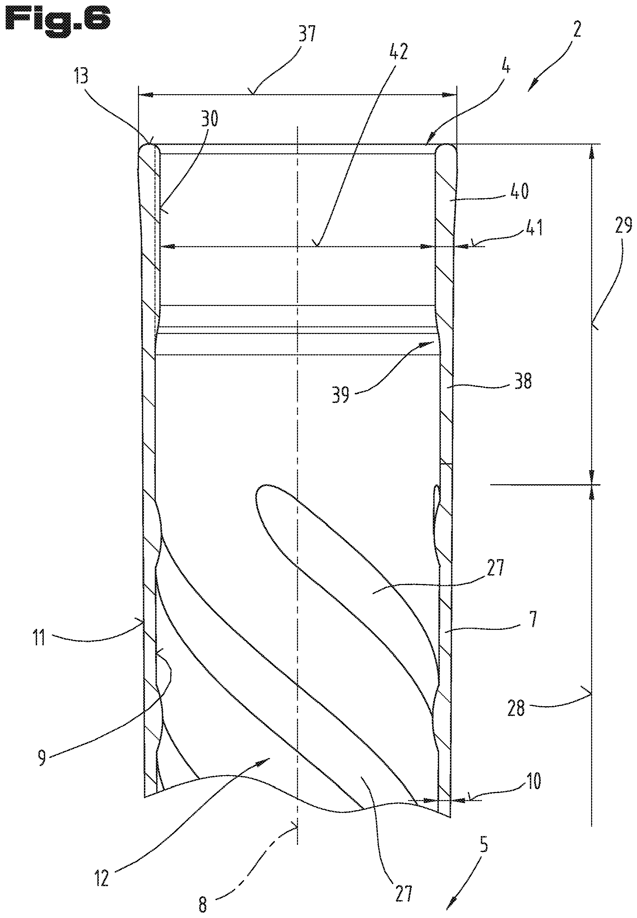

In der Fig. 6 ist eine weitere und gegebenenfalls für sich eigenständige Ausführungsform des Aufnahmebehälters 2 gezeigt, wobei wiederum für gleiche Teile gleiche Bezugszeichen bzw. Bauteilbezeichnungen wie in den vorangegangenen Fig. 1 bis 5 verwendet werden. Um unnötige Wiederholungen zu vermeiden, wird auf die detaillierte Beschreibung in den vorangegangenen Fig. 1 bis 5 hingewiesen bzw. Bezug genommen.

Auch dieser hier gezeigte und beschriebene Aufnahmebehälter 2 weist das offene Ende 4 sowie das verschlossene Ende 5 auf, zwischen welchen sich die Längsachse 8 erstreckt. Im Bereich des ersten Teilabschnitts 28 der Behälterwand 7 ist zumindest einer der zuvor in den Fig. 1 bis 5 beschriebenen Verstärkungsstege 27 über die Innenfläche 9 vorragend angeordnet. Die Wandstärke 10 ist im ersten Teilabschnitts 28 wieder entsprechend reduziert ausgeführt, wie dies ebenfalls zuvor bereits bei den verschiedenen Ausführungsformen und Nenndimensionen beschrieben worden ist.

Der zweite Teilabschnitt 29 ist bei diesem gezeigten Ausführungsbeispiel anders ausgebildet als zuvor beschrieben, wobei diese Ausbildung aber bei all den zuvor beschrieben Ausführungsformen Anwendung finden kann.

Der zuvor beschriebene Nenndurchmesser (entspricht dem äußeren nominellen Durchmesser) ist hier mit dem Bezugszeichen 37 versehen und kann entweder 13 mm oder 16 mm betragen. Um im Dichtabschnitt 30 eine ausreichende Festigkeit bzw. Eigensteifigkeit des offenen Endes 4 zu erzielen und/oder aber ein ungewolltes Eindringen des verschlossenen Endes 5 eines anderen Aufnahmebehälters 2 aufgrund von dessen Konizität hin zum verschlossenen Ende 5 zu vermeiden, ist hier zusätzlich vorgesehen, dass zumindest der Dichtabschnitt 30 über die Innenfläche 9 in Richtung auf die Längsachse 8 vorragend ausgebildet ist. Die Verlängerung der Innenfläche 9 ist in strichlierten Linien im linken Teil des offenen Endes 5 in der Behälterwand 7 eingetragen.

Bei diesem Ausführungsbeispiel ist in einem an den ersten Teilabschnitt 28 anschließenden ersten Wandteil 38 der Behälterwand 7 noch keiner der Verstärkungsstege 27 vorgesehen, wobei sich die reduzierte bevorzugt gleichbleibende Wandstärke 10 in Richtung auf das offenen Ende 4 bis zu einem Übergangsabschnitt 39 erstreckt. Der zum Dichtabschnitt 30 überleitende Übergangsabschnitt 39 kann z.B. durch Übergangsradien und/oder eine oder mehrere Kegelstumpfflächen gebildet sein, wobei aufgrund des in Richtung auf die Längsachse 8 Vorragens im Bereich des Dichtabschnitts 30 ein weiteren Wandteil 40 ausgebildet wird, welcher eine bezüglich des ersten Wandteils 38 dazu größere bzw. dickere Wandstärke 41 aufweist.

Aufgrund der dickeren Ausbildung des weiteren Wandteils 40 und der damit verbundenen Reduzierung der lichten Weite, weist das offene Ende 4 in diesem Axialabschnitt einen Innendurchmesser 42 auf, welcher geringer ist als ein Innendurchmesser in jenem an den Übergangsabschnitt 39 anschließenden ersten Wandteil 38.

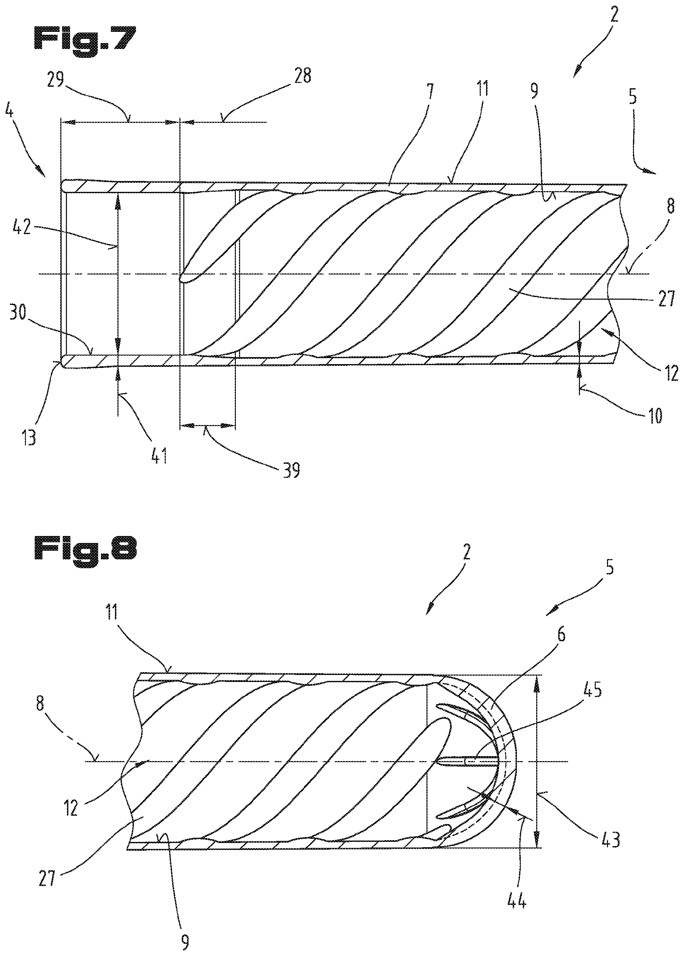

Es wäre aber unabhängig davon auch möglich, dass sich der erste Teilabschnitt 28 bis in den Bereich des Übergangsabschnitts 39 erstreckt und damit der zumindest eine Verstärkungssteg 27 bis unmittelbar an den Übergangsabschnitt 39 heranreicht. Diese mögliche Ausführungsform ist in der nachfolgenden Fig. 7 gezeigt und beschrieben. Die Axialerstreckung des weiteren Wandteils 40 wird bevorzugt so gewählt, dass diese zumindest in etwa der Axialerstreckung der dieser zugewendeten Dichtfläche des Dichtstopfens entspricht.

Der weitere Wandteil 40 bildet durch sein Vorragen beginnend beim Übergangsabschnitt 39 eine Hinterschneidung aus, welche beim Entformen des Formkerns zu überwinden ist.

In der Fig. 7 ist jene Ausbildung und Anordnung des oder der Verstärkungsstege 27 gezeigt, wie dies zuvor bereits angedeutet worden ist. So kann es sich um eine weitere und gegebenenfalls für sich eigenständige Ausführungsform des Aufnahmebehälters 2 handeln, wobei wiederum für gleiche Teile gleiche Bezugszeichen bzw. Bauteilbezeichnungen wie in den vorangegangenen Fig. 1 bis 6 verwendet werden. Um unnötige Wiederholungen zu vermeiden, wird auf die detaillierte Beschreibung in den vorangegangenen Fig. 1 bis 6 hingewiesen bzw. Bezug genommen.

Da diese Ausbildung sehr ähnlich ist, wie diese zuvor in der Fig. 6 bereits näher beschrieben worden ist, wird nur auf die Unterschiede dazu näher eingegangen. Für die unverändert beibehaltenen Ausbildungen wird auf die Beschreibung der Fig. 6 Bezug genommen. Die Ausbildung des verschlossenen Endes 5 kann gemäß einem der hier beschriebenen Ausbildungen erfolgen und frei gewählt werden.

Die Behälterwand 7 weist hier ebenfalls wiederum die zuvor beschriebene geringere Wandstärke 10 auf. Ausgehend vom offenen Ende 4 verjüngt sich die Behälterwand 7 im Bereich ihrer Außenfläche 11 hin zum verschlossenen Ende 5 und endet dort mit einem kalottenförmig ausgebildeten verschlossenen Ende 5. An der Innenfläche 9 sind hier wiederum mehrere der Verstärkungsstege 27 vorgesehen, welche eine in Richtung der Längsachse 8 verlaufende helixförmige Anordnung bzw. Ausbildung aufweisen.

Auch hier ist wiederum vorgesehen, dass im Bereich des Dichtabschnitts 30 die Behälterwand 7 die größere Wandstärke 41 aufweist und sich somit der Innendurchmesser 42 um diesen nach innen vorragenden Wandabschnitt verringert. Der Übergangsabschnitt 39 bildet hier den Übergang zwischen der geringeren Wandstärke 10 sowie der dazu größeren Wandstärke 41 und schließt sich in Axialrichtung direkt an den Dichtabschnitt 30 in Richtung auf das verschlossene Ende 5 daran an. Der Übergangsabschnitt 39 ist in seiner Längserstreckung mit einem Maßpfeil kotiert.

Der erste Teilabschnitt 28 der Behälterwand 7 endet hier in jenem Bereich des Übergangsabschnitts 39, welcher der offenen Stirnseite 13 des Aufnahmebehälters 2 näherliegt. Damit reichen auch die Verstärkungsstege 27 bis nahe oder direkt bis an den Beginn des Dichtabschnitts 30 heran.

Die Fig. 8 zeigt eine weitere und gegebenenfalls für sich eigenständige Ausführungsform des Aufnahmebehälters 2 im Bereich seines verschlossenen Endes 5. Dabei werden wiederum für gleiche Teile gleiche Bezugszeichen bzw. Bauteilbezeichnungen wie in den vorangegangenen Figuren 1 bis 7 verwendet. Um unnötige Wiederholungen zu vermeiden wird auf die detaillierte Beschreibung in den vorangegangenen Figuren 1 bis 7 hingewiesen bzw. Bezug genommen.

Weiters ist hier noch dargestellt, dass der Aufnahmebehälter 2 im Bereich seines verschlossenen Endes 5 im Übergangsbereich zwischen der Bodenwand 6 und der Behälterwand 7 einen Außendurchmesser 43 aufweist. Aufgrund der zuvor beschriebenen Konizität des Aufnahmebehälters 2 ausgehend vom offenen Ende 4 mit dem Nenndurchmesser 37 hin zum verschlossenen Ende 5, ist der Außendurchmesser 43 entsprechend der Konizität kleiner ausgebildet als der Nenndurchmesser 37.

Der zuvor in den Fig. 6 und 7 beschriebene Überstand der verstärkten Behälterwand im Bereich des offenen Endes 4, nämlich im Bereich des Dichtabschnitts 30, ist derart zu wählen, dass der Innendurchmesser 42 im Bereich des Dichtabschnitts 30 kleiner gewählt ist als der Außendurchmesser 43 im Bereich des verschlossenen Endes 5. Damit kann ein Eindringen des verschlossenen Endes 5 in das offene Ende 4 bei lose nebeneinander angeordneten Aufnahmebehältern 2 und somit ein gegenseitiges Verklemmen von Aufnahmebehältern 2 bzw. eine Stangenbildung derselben verhindert werden.

Die Bodenwand 6 weist bei diesem Ausführungsbeispiel eine Bodenwandstärke 44 auf, welche in etwa der geringeren Wandstärke 10 der Behälterwand 7 entspricht. Durch die Reduzierung der Wandstärke 10, insbesondere auch der Bodenwandstärke 44, kann es im Zuge der beim Zentrifugiervorgang des Aufnahmebehälters 2 bzw. der Aufnahmeeinheit 1 auftretenden hohen Belastungen zu ungewollten Verformungen der Bodenwand 6 kommen. Diese könnte in Richtung auf den Aufnahmeraum 12 hinein gedrückt werden.

Zur Vermeidung bzw. Verhinderung von derartigen ungewollten Verformungen und einem möglichen damit einhergehenden ungewollten Austritt von im Aufnahmeraum 12 bevorrateten und zu trennenden Medien ist hier noch vorgesehen, dass im Bereich der Innenfläche 9 der Bodenwand 6 ausgehend von der Bodenwand 6 in Richtung auf den Aufnahmeraum 12 vorragende Stege 45 angeordnet bzw. vorgesehen sein können. Bevorzugt werden mehrere derartige Stege 45 über den Umfang verteilt an der Bodenwand 6 angeordnet bzw. ausgebildet. Die Anordnung der einzelnen Stege 45 zueinander kann unterschiedlichst erfolgen, wobei hier eine sternförmige Anordnung ausgehend von einem durch die Längsachse 8 gebildeten Zentrum der Bodenwand 6 hin zur umlaufenden Behälterwand 7 gewählt worden ist.

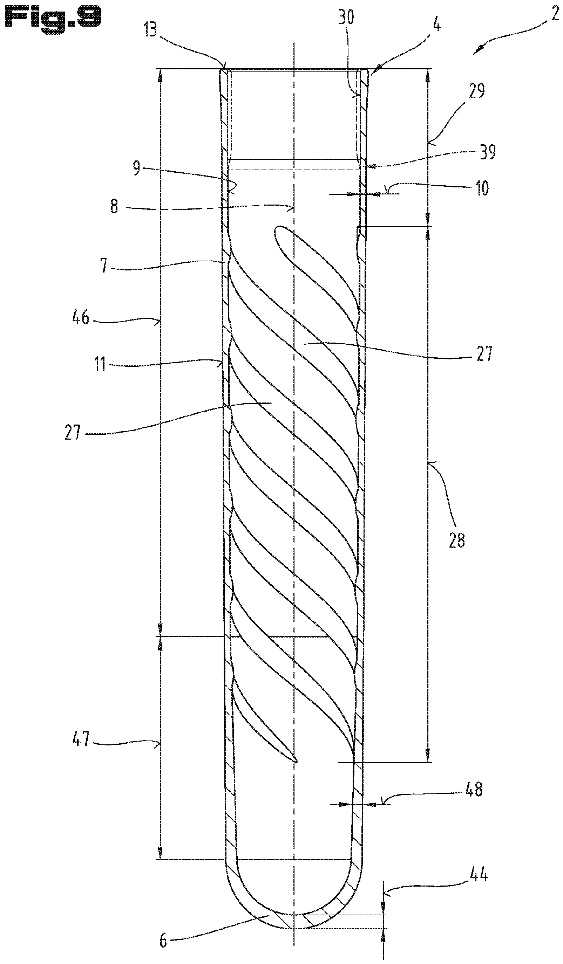

In der Fig. 9 ist eine weitere mögliche und gegebenenfalls für sich eigenständige Ausbildung des Aufnahmebehälters 2 zur Bildung der Aufnahmeeinheit 1 gezeigt, wobei wiederum für gleiche Teile gleiche Bezugszeichen bzw. Bauteilbezeichnungen wie in den vorangegangenen Fig. 1 bis 8 verwendet werden. Um unnötige Wiederholungen zu vermeiden, wird auf die detaillierte Beschreibung in den vorangegangenen Fig. 1 bis 8 hingewiesen bzw. Bezug genommen.

Die Behälterwand 7 weist ausgehend vom offenen Ende 4 mit seiner Stirnseite 13 in Richtung auf das verschlossene Ende 5 hin in etwa die gleiche geringe Wandstärke 10 auf, wie dies bereits zuvor beschrieben worden ist. Diese Axialerstreckung bzw. die Längserstreckung des Wandabschnitts mit der geringeren Wandstärke 10 weist einen Wert auf, welcher in etwa 2/3 der Nennlänge des Aufnahmebehälters 2 entspricht. Dieser Längsabschnitt der Behälterwand 7 kann auch als Basislängsabschnitt 46 bezeichnet werden und ist mit einer Längskotierung versehen. Zwischen dem dem verschlossenen Ende 5 zugewendeten Ende des Basislängsabschnitts 46 und der Bodenwand 6 ist ein bodenseitiger Längsabschnitt 47 an der Behälterwand 7 ausgebildet. Dieser bodenseitige Längsabschnitt 47 der Behälterwand 7 weist eine in Richtung auf das verschlossene Ende 5 nach innen hin zunehmende Wanddicke 48 auf, wodurch die Innenabmessung bzw. der Innendurchmesser des Aufnahmeraums 12 in dem bodenseitigen Längsabschnitt 47 sich rascher verkleinert als im Bereich des Basislängsabschnitts 46. Die Zunahme der Wanddicke 48 kann stetig bzw. linear erfolgen.

Im Axialschnitt gesehen bildet die Außenfläche 11 der Behälterwand 7 eine Gerade aus, welche zusätzlich bezüglich der Längsachse 8 eine entsprechende Konizität aufweist. Im Bereich des offenen Endes 4 kann die Behälterwand 7 im Bereich ihrer Außenfläche 11 eine geringfügig stärkere bzw. größere Wandstärke 10 aufweisen, wie dies im Bereich des Dichtabschnitts 30 zu ersehen ist.

Durch die Zunahme der Wanddicke 48 der Behälterwand 7 im Bereich des bodenseitigen Längsabschnitts 47 kann auch die Bodenwand 6 mit der dazu entsprechenden Bodenwandstärke 44 ausgebildet werden. Dabei kann die Bodenwandstärke 44 durchgängig jener Wandstärke entsprechen, wie diese bei bislang bekannten Aufnahmebehältern 2 ausgebildet worden ist. So kann die Bodenwandstärke 44 einen Wert mit einer unteren Grenze von 0,8 mm und einer oberen Grenze von 1,2 mm, bevorzugt 1,0 mm, aufweisen.

An der Innenfläche 9 der Behälterwand 7 können wiederum die zuvor beschriebenen Verstärkungsstege 27 angeordnet bzw. ausgebildet sein. In Axialrichtung gesehen endet der oder enden die Verstärkungssteg(e) 27 noch vor dem Dichtabschnitt 30, wie dies bereits in der Fig. 6 mit dem zweiten Teilabschnitt 29 gezeigt und beschrieben ist. Der erste Teilabschnitt 28, in welchem der oder die Verstärkungsstege 27 angeordnet bzw. vorgesehen sind, reicht ausgehend vom zweiten Teilabschnitt 29 hin in Richtung auf das verschlossene Ende 5 bis in etwa die Hälfte der Längserstreckung des bodenseitigen Längsabschnitts 47.

Durch diese zuvor beschriebenen Ausbildungen mit den in Richtung der Längsachse 8 zueinander unterschiedlichen Wandstärken bzw. Wanddicken der Behälterwand 7 und/oder der Bodenwand 6 sowie der zumindest bereichsweisen Anordnung des oder der Verstärkungsstege 27 und/oder der Stege 45 an der Bodenwand 6 kann so ein auf den jeweiligen Einsatzzweck abgestimmter Aufnahmebehälter 2 geschaffen werden. Damit kann an vorbestimmten Stellen bzw. vorbestimmten Längsabschnitten mit einer geringeren Wandstärke 10 zur Einsparung von Rohstoffressourcen das Auslangen gefunden werden.

Um auch bei den reduzierten Wandstärken 10, wie dies für alle erfindungsgemäßen Aufnahmebehälter 2 zuvor beschrieben worden ist, kann dem Kunststoffwerkstoff zumindest ein Barriereadditiv ausgewählt aus der Gruppe von anorganischen Magnesium-Aluminiumsilikate, Ethylen-Vinylalkohol-Copolymere (EVOH), Polyvinylidenchlorid (PVDC), Masterbatch bzw. Scavenger zur Verbesserung der Gasbarriereeigenschaften zugesetzt sein. Unabhängig davon oder zusätzlich dazu wäre es aber auch noch möglich, dass zumindest an der Innenfläche 9 der Behälterwand 7 eine Sperrbeschichtung wie z.B. aus dem Werkstoff SiOx angeordnet bzw. aufgebracht ist. Diese Sperrbeschichtung kann aber auch auf der Außenfläche 11 oder aber auch auf beiden der Flächen (Innenfläche 9 sowie Außenfläche 11) angeordnet bzw. aufgebracht sein.

Sämtliche Angaben zu Wertebereichen in gegenständlicher Beschreibung sind so zu verstehen, dass diese beliebige und alle Teilbereiche daraus mitumfassen, z.B. ist die Angabe 1 bis 10 so zu verstehen, dass sämtliche Teilbereiche, ausgehend von der unteren Grenze 1 und der oberen Grenze 10 mit umfasst sind, d.h. sämtliche Teilbereiche beginnen mit einer unteren Grenze von 1 oder größer und enden bei einer oberen Grenze von 10 oder weniger, z.B. 1 bis 1,7, oder 3,2 bis 8,1, oder 5,5 bis 10.

Der Ordnung halber sei abschließend darauf hingewiesen, dass zum besseren Verständnis des Aufbaus der Aufnahmeeinheit 1 diese bzw. deren Bestandteile teilweise unmaßstäblich und/oder vergrößert und/oder verkleinert dargestellt wurden.

**Bezugszeichenaufstellung**

| | | | |
|---|---|---|---|
| 1 | Aufnahmeeinheit | 31 | Breite |
| 2 | Aufnahmebehälter | 32 | Überstand |
| 3 | Verschlussvorrichtung | 33 | Verstärkungssteg |
| 4 | offenes Ende | 34 | Übergangsstufe |
| 5 | verschlossenes Ende | 35 | Stirnfläche |
| 6 | Bodenwand | 36 | Übergangsabschnitt |
| 7 | Behälterwand | 37 | Nenndurchmesser |
| 8 | Längsachse | 38 | Wandteil |
| 9 | Innenfläche | 39 | Übergangsabschnitt |
| 10 | Wandstärke | 40 | Wandteil |
| 11 | Außenfläche | 41 | Wandstärke |
| 12 | Aufnahmeraum | 42 | Innendurchmesser |
| 13 | Stirnseite | 43 | Außendurchmesser |
| 14 | Kappe | 44 | Bodenwandstärke |
| 15 | Dichtstopfen | 45 | Steg |
| 16 | Kappenmantel | 46 | Basislängsabschnitt |
| 17 | Fortsatz | 47 | Längsabschnitt |
| 18 | Fortsatz | 48 | Wanddicke |
| 19 | Haltering | | |
| 20 | Ansatz | | |
| 21 | Dichtfläche | | |
| 22 | Dichtfläche | | |
| 23 | Vertiefung | | |
| 24 | Öffnung | | |
| 25 | Endbereich | | |
| 26 | Endbereich | | |
| 27 | Verstärkungssteg | | |
| 28 | erster Teilabschnitt | | |
| 29 | zweiter Teilabschnitt | | |
| 30 | Dichtabschnitt | | |

## Patentansprüche

1. Aufnahmeeinheit (1) zur Aufnahme von Blut, umfassend einen als Blutprobenröhrchen ausgebildeten Aufnahmebehälter (2), umfassend ein offenes Ende (4), ein mit einer Bodenwand (6) verschlossenes Ende (5), eine überwiegend rohrförmig ausgebildete Behälterwand (7), welche sich zwischen dem offenen Ende (4) und dem verschlossenen Ende (5) erstreckt und eine Längsachse (8) definiert, wobei die Behälterwand (7) eine Innenfläche (9) sowie eine in einer Wandstärke (10) davon distanzierte Außenfläche (11) aufweist, und die Behälterwand (7) sowie das mit der Bodenwand (6) verschlossene Ende (5) einen Aufnahmeraum (12) umgrenzen, in welchem Aufnahmeraum (12) das Blut unmittelbar aufnehmbar ist, und wobei der Aufnahmebehälter (2) aus einem Kunststoffwerkstoff gebildet ist, wobei zumindest an einem ersten Teilabschnitt (28) der Innenfläche (9) der Behälterwand (7) zumindest ein in Richtung auf die Längsachse (8) vorragender Verstärkungssteg (27) angeordnet ist, wobei sich der erste Teilabschnitt (28) ausgehend vom verschlossenen Ende (5) hin in Richtung auf das offene Ende (4) erstreckt, und wobei der zumindest eine Verstärkungssteg (27) einen wendelförmigen, insbesondere helixförmigen, Längsverlauf um die Längsachse (8) aufweist, wobei die Aufnahmeeinheit (1) weiters eine Verschlussvorrichtung (3) umfasst, mit welcher das offene Ende (4) des Aufnahmebehälters (2) verschlossen ist, **dadurch gekennzeichnet, dass** ein gegenüber der äußeren Umgebungsatmosphäre abgeschlossener Aufnahmeraum (12) auf einen bezüglich des Umgebungsdruckes dazu reduzierten Druck abgesenkt ist und dass die Wandstärke (10) der Behälterwand (7) einen Wert aufweist, der aus einem Wertebereich stammt, dessen untere Grenze 0,3 mm und dessen obere Grenze 0,8 mm beträgt und ein Überstand (32) des zumindest einen Verstärkungsstegs (27) über die Innenfläche (9) einen Wert aufweist, der aus einem Wertebereich stammt, dessen untere Grenze 0,05 mm und dessen obere Grenze 0,60 mm beträgt und eine Breite (31) des zumindest einen Verstärkungsstegs (27) in senkrechter Richtung bezüglich seiner Längserstreckung einen Wert aufweist, der aus einem Wertebereich stammt, dessen untere Grenze 0,50 mm und dessen obere Grenze 4,00 mm beträgt und die Bodenwand (6) kalottenförmig ist.

2. Aufnahmeeinheit (1) zur Aufnahme von Blut nach Anspruch 1, **dadurch gekennzeichnet, dass** mehrere über den Umfang gleichmäßig verteilt angeordnete Verstärkungsstege (27) vorgesehen sind.

3. Aufnahmeeinheit (1) zur Aufnahme von Blut nach Anspruch 1 oder 2, **dadurch gekennzeichnet, dass** eine Steigung des zumindest einen Verstärkungsstegs (27) bezogen auf einen vollen Umfang von 360° einen Wert aufweist, der aus einem Wertebereich stammt, dessen untere Grenze 20 mm und dessen obere Grenze 60 mm beträgt.

4. Aufnahmeeinheit (1) zur Aufnahme von Blut nach einem der vorhergehenden Ansprüche, **dadurch gekennzeichnet, dass** ein Außendurchmesser des Blutprobenröhrchen im Bereich seines offenen Endes (4) einen Wert aufweist, der aus einem Wertebereich stammt, dessen untere Grenze 12 mm und dessen obere Grenze 18 mm beträgt.

5. Aufnahmeeinheit (1) zur Aufnahme von Blut nach einem der vorhergehenden Ansprüche, **dadurch gekennzeichnet, dass** eine Gesamtlänge des Blutprobenröhrchens in Richtung seiner Längsachse (8) einen Wert aufweist, der aus einem Wertebereich stammt, dessen untere Grenze 65 mm und dessen obere Grenze 130 mm beträgt.

6. Aufnahmeeinheit (1) zur Aufnahme von Blut nach einem der vorhergehenden Ansprüche, **dadurch gekennzeichnet, dass** der zumindest eine Verstärkungssteg (27) ausschließlich im ersten Teilabschnitt (28) der Innenfläche (9) der Behälterwand (7) angeordnet ist.

7. Aufnahmeeinheit (1) zur Aufnahme von Blut nach einem der vorhergehenden Ansprüche, **dadurch gekennzeichnet, dass** ein zweiter Teilabschnitt (29) der Innenfläche (9) der Behälterwand (7) anschließend an den ersten Teilabschnitt (28) vorgesehen ist, welcher zweite Teilabschnitt (29) zumindest bereichsweise einen in etwa zylindrisch ausgebildeten Dichtabschnitt (30) ausbildet.

8. Aufnahmeeinheit (1) zur Aufnahme von Blut nach Anspruch 7, **dadurch gekennzeichnet, dass** zumindest der Dichtabschnitt (30) über die Innenfläche (9) der Behälterwand (7) in Richtung auf die Längsachse (8) vorragend ausgebildet ist, wobei ein Wandteil (40) der Behälterwand (7) im Bereich des Dichtabschnitts (30) eine bezüglich der Wandstärke (10) des ersten Teilabschnitts (28) der Behälterwand (7) eine dazu größere Wandstärke (41) aufweist.

9. Aufnahmeeinheit (1) zur Aufnahme von Blut nach einem der vorhergehenden Ansprüche, **dadurch gekennzeichnet, dass** an der Bodenwand (6) an ihrer dem Aufnahmeraum (12) zugewendeten Innenfläche zumindest ein über deren Innenfläche vorragend ausgebildeter Steg (45) angeordnet ist.

10. Aufnahmeeinheit (1) zur Aufnahme von Blut nach einem der vorhergehenden Ansprüche, **dadurch gekennzeichnet, dass** die Bodenwand (6) eine bezüglich der Wandstärke (10) der Behälterwand (7) dazu größere Bodenwandstärke (44) aufweist.

11. Aufnahmeeinheit (1) zur Aufnahme von Blut nach einem der vorhergehenden Ansprüche, **dadurch gekennzeichnet, dass** die Behälterwand (7) in einem bodenseitigen Längsabschnitt (47) eine bezüglich der übrigen Wandstärke (10) hin in Richtung auf die Bodenwand (6) dazu zunehmende Wanddicke (48) aufweist.

12. Aufnahmeeinheit (1) zur Aufnahme von Blut nach einem der vorhergehenden Ansprüche, **dadurch gekennzeichnet, dass** der Kunststoffwerkstoff ausgewählt ist aus der Gruppe von Polyethylenterephthalat (PET), Polyethylen (PE), Polypropylen (PP), Polystyrol (PS), Polycarbonat (PC), High-Density-Polyethylen (PE-HD), Acrylnitril-Butadien-Styrol-Copolymere (ABS), Polyamid (PA.

13. Aufnahmeeinheit (1) zur Aufnahme von Blut nach einem der vorhergehenden Ansprüche, **dadurch gekennzeichnet, dass** zumindest an der Innenfläche (9) und/oder der Außenfläche (11) der Behälterwand (7) eine Sperrbeschichtung, insbesondere aus dem Werkstoff SiOx , angeordnet oder aufgebracht ist.

## Claims

1. A receiving unit (1) for receiving blood, comprising a receiving container (2) designed as a blood sample tube, comprising an open end (4), an end (5) closed by a base wall (6), a predominantly tube-shaped container wall (7) which extends between the open end (4) and the closed end (5) and defines a longitudinal axis (8), wherein the container wall (7) comprises an internal surface (9) and an external surface (11) spaced apart therefrom by a wall thickness (10), and the container wall (7) and the end (5) closed by the base wall (6) bound a receiving chamber (12), in which receiving chamber (12) the blood can be directly received, and wherein the receiving container (2) is made from a plastic material, wherein at least one reinforcement rib (27) is arranged on at least one first subsection (28) of the internal surface (9) of the container wall (7) and protrudes in the direction towards the longitudinal axis (8), wherein the first subsection (28) extends from the closed end (5) in the direction towards the open end (4), and wherein the at least one reinforcement rib (27) has a spiral-shaped, in particular helical, longitudinal extension about the longitudinal axis (8), wherein the receiving unit (1) further comprises a closure device (3) by means of which the open end (4) of the receiving container (2) is closed, **characterized in that** a receiving chamber (12) sealed off from the external ambient atmosphere is reduced to a pressure below ambient pressure and that the wall thickness (10) of the container wall (7) has a value selected from a range of values with a lower limit of 0.3 mm and an upper limit of 0.8 mm and a protrusion (32) of the at least one reinforcement rib (27) beyond the internal surface (9) has a value selected from a range of values with a lower limit of 0.05 mm and an upper limit of 0.60 mm and a width (31) of the at least one reinforcement rib (27) in the direction perpendicular to its longitudinal extension has a value selected from a range of values with a lower limit of 0.50 mm and an upper limit of 4.00 mm and the base wall (6) is calotte-shaped.

2. The receiving unit (1) for receiving blood according to claim 1, **characterized in that** several reinforcement ribs (27) are provided, distributed uniformly across the circumference.

3. The receiving unit (1) for receiving blood according to claim 1 or 2, **characterized in that** a pitch of the at least one reinforcement rib (27) relative to a full circumference of 360° has a value selected from a range of values with a lower limit of 20 mm and an upper limit of 60 mm.

4. The receiving unit (1) for receiving blood according to one of the preceding claims, **characterized in that** an external diameter of the blood sample tube in the region of its open end (4) has a value selected from a range of values with a lower limit of 12 mm and an upper limit of 18 mm.

5. The receiving unit (1) for receiving blood according to one of the preceding claims, **characterized in that** a total length of the blood sample tube in the direction of its longitudinal axis (8) has a value selected from a range of values with a lower limit of 65 mm and an upper limit of 130 mm.

6. The receiving unit (1) for receiving blood according to one of the preceding claims, **characterized in that** the at least one reinforcement rib (27) is disposed exclusively in the first subsection (28) of the internal surface (9) of the container wall (7).

7. The receiving unit (1) for receiving blood according to one of the preceding claims, **characterized in that** a second subsection (29) of the internal surface (9) of the container wall (7) is disposed adjoining the first subsection (28), which second subsection (29) is designed as an approximately cylindrical sealing section (30) in at least certain regions.

8. The receiving unit (1) for receiving blood according to claim 7, **characterized in that** at least the sealing section (30) projects beyond the internal surface (9) of the container wall (7) in the direction towards the longitudinal axis (8), wherein a wall part (40) of the container wall (7) in the region of the sealing section (30) has a bigger wall thickness (41) than the wall thickness (10) of the first subsection (28) of the container wall (7).

9. The receiving unit (1) for receiving blood according to one of the preceding claims, **characterized in that** at least one web (45) is provided on the base wall (6) on its internal surface facing the receiving chamber (12) and protruding from the internal surface thereof.

10. The receiving unit (1) for receiving blood according to one of the preceding claims, **characterized in that** the base wall (6) has a bigger base wall thickness (44) than the wall thickness (10) of the container wall (7).

11. The receiving unit (1) for receiving blood according to one of the preceding claims, **characterized in that** the container wall (7) at a base-end longitudinal section (47) has a wall thickness (48) that increases in the direction towards the base wall (6) relative to the rest of the wall thickness (10).

12. The receiving unit (1) for receiving blood according to one of the preceding claims, **characterized in that** the plastic material is selected from the group comprising polyethylene terephthalate (PET), polyethylene (PE), polypropylene (PP), polystyrene (PS), polycarbonate (PC), high-density polyethylene (HD-PE), acrylonitrile-butadiene-styrene copolymers (ABS), polyamides (PA).

13. The receiving unit (1) for receiving blood according to one of the preceding claims, **characterized in that** a barrier coating, in particular from the material SiOX, is arranged on or applied to at least the internal surface (9) and/or the external surface (11) of the container wall (7).

## Revendications

1. Unité de réception (1) destinée à recevoir du sang, comprenant un récipient de réception (2) constitué en tant que tube pour échantillons de sang, comprenant une extrémité ouverte (4), une extrémité fermée (5) avec une paroi de base (6), une paroi de récipient (7) constituée principalement sous forme tubulaire qui s'étend entre l'extrémité ouverte (4) et l'extrémité fermée (5) et définit un axe longitudinal (8), la paroi de récipient (7) comportant une surface intérieure (9) ainsi qu'une surface extérieure (11) qui en est distante dans une épaisseur de paroi (10), et la paroi de récipient (7) ainsi que l'extrémité fermée (5) fermée avec la paroi de base (6) délimitant un espace de réception (12), dans lequel espace de réception (12) le sang peut être reçu directement, et le récipient de réception (2) étant formé d'une matière plastique, au moins une âme de renforcement (27) en saillie en direction de l'axe longitudinal (8) étant disposée au moins sur un premier segment partiel (28) de la surface intérieure (9) de la paroi de récipient (7), le premier segment partiel (28) s'étendant en partant de l'extrémité fermée (5) en direction de l'extrémité ouverte (4), et l'âme de renforcement (27) au moins au nombre de un présentant un profil longitudinal en spirale, en particulier en forme d'hélice, autour de l'axe longitudinal (8), l'unité de réception (1) comprenant en outre un dispositif de fermeture (3) avec lequel l'extrémité ouverte (4) du récipient de réception (2) est fermée, **caractérisée en ce qu'**un espace de réception (12) fermé vis-à-vis de l'atmosphère ambiante extérieure est abaissé à une pression réduite par rapport à la pression ambiante, et **en ce que** l'épaisseur de paroi (10) de la paroi de récipient (7) présente une valeur qui s'inscrit dans une plage de valeurs dont la limite inférieure est de 0,3 mm et dont la limite supérieure est de 0,8 mm, et un surplomb (32) de l'âme de renforcement (27) au moins au nombre de un au-dessus de la surface intérieure (9) présente une valeur qui s'inscrit dans une plage de valeurs dont la limite inférieure est de 0,05 mm et dont la limite supérieure est de 0,60 mm, et une largeur (31) de l'âme de renforcement (27) au moins au nombre de un présente, dans la direction perpendiculaire par rapport à son étendue longitudinale, une valeur qui s'inscrit dans une plage de valeurs dont la limite inférieure est de 0,50 mm et dont la limite supérieure est de 4,00 mm, et la paroi de base (6) est en forme de calotte.

2. Unité de réception (1) destinée à recevoir du sang selon la revendication 1, **caractérisée en ce qu'**il prévu plusieurs âmes de renforcement (27) disposées de façon uniformément répartie sur la circonférence.

3. Unité de réception (1) destinée à recevoir du sang selon la revendication 1 ou 2, **caractérisée en ce qu'**une pente de l'âme de renforcement (27) au moins au nombre de un présente, par rapport à une pleine circonférence de 360°, une valeur qui s'inscrit dans une plage de valeurs dont la limite inférieure est de 20 mm et dont la limite supérieure est de 60 mm.

4. Unité de réception (1) destinée à recevoir du sang selon l'une des revendications précédentes, **caractérisée en ce qu'**un diamètre extérieur du tube pour échantillons de sang présente, dans la zone de son extrémité ouverte (4), une valeur qui s'inscrit dans une plage de valeurs dont la limite inférieure est de 12 mm et dont la limite supérieure est de 18 mm.

5. Unité de réception (1) destinée à recevoir du sang selon l'une des revendications précédentes, **caractérisée en ce qu'**une longueur totale du tube pour échantillons de sang présente, en direction de son axe longitudinal (8), une valeur qui s'inscrit dans une plage de valeurs dont la limite inférieure est de 65 mm et dont la limite supérieure est de 130 mm.

6. Unité de réception (1) destinée à recevoir du sang selon l'une des revendications précédentes, **caractérisée en ce que** l'âme de renforcement (27) au moins au nombre de un est disposée exclusivement dans le premier segment partiel (28) de la surface intérieure (9) de la paroi de récipient (7).

7. Unité de réception (1) destinée à recevoir du sang selon l'une des revendications précédentes, **caractérisée en ce qu'**un deuxième segment partiel (29) de la surface intérieure (9) de la paroi de récipient (7) est prévu à la suite du premier segment partiel (28), lequel deuxième segment partiel (29) constitue au moins partiellement un segment d'étanchéité (30) constitué de façon à peu près cylindrique.

8. Unité de réception (1) destinée à recevoir du sang selon la revendication 7, **caractérisée en ce qu'**au moins le segment d'étanchéité (30) est constitué en saillie au-dessus de la surface intérieure (9) de la paroi de récipient (7) en direction de l'axe longitudinal (8), une partie de paroi (40) de la paroi de récipient (7) présentant, dans la zone du segment d'étanchéité (30), une épaisseur de paroi (41) plus grande par rapport à l'épaisseur de paroi (10) du premier segment partiel (28) de la paroi de récipient (7).

9. Unité de réception (1) destinée à recevoir du sang selon l'une des revendications précédentes, **caractérisée en ce que**, sur la paroi de base (6), sur sa surface intérieure tournée vers l'espace de réception (12), il est disposé au moins une âme (45) constituée en saillie au-dessus de sa surface intérieure.

10. Unité de réception (1) destinée à recevoir du sang selon l'une des revendications précédentes, **caractérisée en ce que** la paroi de base (6) présente une épaisseur de paroi de fond (44) plus grande par rapport à l'épaisseur de paroi (10) de la paroi de récipient (7).

11. Unité de réception (1) destinée à recevoir du sang selon l'une des revendications précédentes, **caractérisée en ce que**, dans un segment longitudinal (47) côté base, la paroi de récipient (7) présente une épaisseur de paroi (48) qui augmente rapport au reste de l'épaisseur de paroi (10) en direction de la paroi de base (6).

12. Unité de réception (1) destinée à recevoir du sang selon l'une des revendications précédentes, **caractérisée en ce que** la matière plastique est sélectionnée dans le groupe composé de polyéthylène téréphtalate (PET), de polyéthylène (PE), de polypropylène (PP), de polystyrène (PS), de polycarbonate (PC), de polyéthylène haute densité (PE-HD), de copolymère acrylonitrile-butadiène-styrène (ABS), de polyamide (PA).

13. Unité de réception (1) destinée à recevoir du sang selon l'une des revendications précédentes, **caractérisée en ce que**, au moins sur la surface intérieure (9) et/ou la surface extérieure (11) de la paroi de récipient (7), il est disposé ou appliqué un revêtement barrière, en particulier en matériau SiOx.
